# EUROPEAN PATENT APPLICATION

(11) **EP 4 596 572 A1**
(43) Date of publication of application: **06.08.2025**
(21) Application number: 23873072.5
(22) Date of filing: 26.09.2023
(51) Int. Cl.: C07K 7/06, C07K 7/08, C07K 14/00, C07K 1/34

(54) **FUSION PROTEIN FORMING SELF-ASSEMBLY INCLUDING AMORPHOUS PEPTIDE TAG WITH MAIN AMINO ACIDS COMPOSED OF CHARGED AND HYDROPHOBIC AMINO ACIDS, AND METHOD FOR PURIFYING RECOMBINANT PROTEINS USING SAME**

(30) Priority: 27.09.2022 KR 20220122187
(71) Applicant: CCRIPO Inc., Suncheon-si, Jeollanam-do 57922 (KR)
(72) Inventor: KIM, Dae Heon, Suncheon-si, Jeollanam-do 57930 (KR); NA, Yun Jeong, Suncheon-si, Jeollanam-do 57930 (KR); KIM, Jonghak, Sangju-si, Gyeongsangbuk-do 37137 (KR); CHOI, Seon, Gwangyang-si, Jeollanam-do 57784 (KR)
(74) Representative: Reddie & Grose LLP
(86) International application number: PCT/KR2023/014763
(87) International publication number: WO 2024/071957

(57) **Abstract**

The present invention relates to an amorphous peptide tag with main amino acids composed of charged and hydrophobic amino acids, a fusion protein in which the tag is fused with a target protein, and a method for purifying the target protein using same. Since the peptide tag can induce the formation of self-assemblies when fused with the target protein, the method for purifying the target protein utilizing same allows for the separation and purification of the target protein only using very simple methods such as centrifugation or filtration.

## Description

### [TECHNICAL FIELD]

### Cross-reference to related application(s)

The present application claims the benefit of the priority based on Korean Patent Application No. 10-2022-0122187 filed on September 27, 2022, and the entire contents disclosed in the documents of the corresponding Korean patent application are incorporated as a part of the present description. The present invention relates to a fusion protein comprising an amorphous (disordered, unstructured) peptide tag in which the major amino acids are composed of charged and hydrophobic amino acids, a composition for purifying a recombinant protein comprising the same, and a method for purifying a recombinant protein using thereof.

### [BACKGROUND ART]

In general, His tag (or His₆ tag) or HAT tag (natural histidine affinity tag) together with MBP (maltose-binding protein) tag, GST(glutathione s-transferase) tag are widely used for purification of recombinant proteins. A generalized method for purification of recombinant proteins using a His tag, purifies them by a method of fusing a His tag to a recombinant protein to bind it to a Ni²⁺-NTA bead (or resin) or Co²⁺-CMA bead in which a nickel ion or cobalt ion is immobilized through affinity chromatography, and then eluting the recombinant protein bound to the bead through an excessive amount of imidazole or low pH treatment. In other words, it is to elute a recombinant protein fused with a His tag using the principle that imidazole with a similar structure to amino acid histidine competes with a His tag and binds to a Ni²⁺-NTA bead or Co²⁺-CMA bead (Kerpe K. 2003). Like this, the most widely used method for purification of recombinant proteins including protein pharmaceuticals is adsorption (affinity) chromatography using an affinity tag or using protein A having binding affinity to an antibody. However, such purification methods have various problems, such that they should pass through a time-consuming, complex multistep purification process, and the purification purity is lowered a little due to contamination of proteins having non-specific binding capacity to beads, and a process of removing a tag with a big size is needed after the purification, and it is difficult to use recycled beads for production of other types of recombinant proteins, and the like. These problems act as major factors that limit scale-up of the purification step and increase production costs.

On the other hand, many studies have been conducted on motifs, domains, or peptides that can nucleation or formation of multimers such as dimers. It has been reported that a trimer dependent on metal ions of Ni²⁺, Zn²⁺, Cu²⁺ is formed by artificially inducing a histidine residue in an isoleucine zipper peptide forming a coil structure. In addition, it is known that the TZ1H peptide having a coil structure also forms a trimer through Ag²⁺ or pH-dependent structure conversion. Furthermore, it has been reported that ferritin that is an iron storage protein discovered in various species has ability to bind to an iron ion, and 24 ferritin proteins are combined to form one ferritin complex. Moreover, it has been known that alpha-helical (α-helical) peptide 18A, beta-strand (β-strand) peptide ELK16. Amphipathic surfactant-like peptide L₆KD, and hydrophobic peptide GFIL8 and the like form inclusion bodies in cells. In addition, it has been reported that ELPs (Elastin like polypeptides) undergo protein aggregation, and studies to apply this characteristic to purification of recombinant proteins have been conducted, and it has been reported that calmodulin and M13 peptide bind specifically in a calcium ion-dependent manner, the binding capacity between Atox1 and WD4 is dependent on Zn²⁺, Cu²⁺, Pt²⁺, and FRB (FKBP12-Rapamycin binding protein) and FKBP (FK506 binding protein) bind specifically under the presence of rapamycin.

Under the characteristic background of the motif, domain or peptide of the technical or specific protein aforementioned, the present inventors have completed the present invention which can solve problems of conventional methods for purifying recombinant proteins, and can selectively purify high purity recombinant proteins by a simple method such as centrifugation or filtration by inducing macromolecular(supramolecular) assembly formation through specific inducer or condition treatment that is a completely different concept from the conventional methods.

### [DISCLOSURE]

### [TECHNICAL PROBLEM]

The technical problem to be achieved by the present invention is to solve various problems such as use of expensive beads or resins, requirement of an expensive device or equipment operated for purification, consumption of a lot of time, and undergoing of a multistep complex process, and the like, in conventional recombinant protein purification methods, namely, purification processes, and relates to a method for separating and purifying a targeted recombinant protein only by a simple method such as centrifugation, filtration, or the like, by inducing formation of a macromolecular self-assembly of a tag fused to a recombinant protein in a specific inducer or condition dependent manner. Through this, it is to develop a competitive next-generation method for purifying a recombinant protein that does not require expensive beads or resins and can purity recombinant proteins very easily, rapidly, simply, with high purity, high efficiency and low costs.

One example of the present disclosure provides an amorphous tag that forms a self-assembly, in which the major amino acids are composed of charged and hydrophobic amino acids.

Another embodiment of the present application provides a fusion polypeptide comprising a first polypeptide represented by SEQ ID NO: 1, and a second polypeptide which is a peptide tag in which major amino acids are composed of charged and hydrophobic amino acids.

Other embodiment of the present application provides a fusion protein comprising a fusion polypeptide comprising the first polypeptide and the second polypeptide and a target protein.

Other embodiment of the present application provides a polynucleotide encoding a fusion polypeptide comprising the first polypeptide and the second polypeptide, or a fusion protein comprising the fusion polypeptide and a target protein.

Other embodiment of the present application provides an expression vector comprising a polynucleotide encoding a fusion polypeptide comprising the first polypeptide and the second polypeptide, or a fusion protein comprising the fusion polypeptide and a target protein.

Other embodiment of the present application provides a host cell transformed with the expression vector.

Other embodiment of the present application provides a method for purifying a target protein comprising culturing a cell comprising a polynucleotide encoding a fusion polypeptide comprising the first polypeptide and the second polypeptide, or a fusion protein comprising the fusion polypeptide and a target protein, in a medium.

Other embodiment of the present application provides a composition for purifying a protein comprising a first polypeptide represented by SEQ ID NO: 1, and/or a second polypeptide represented by one amino acid sequence selected from the group consisting of SEQ ID NO: 2 to SEQ ID NO: 5.

Other embodiment of the present application provides a self-assembly comprising a first polypeptide represented by SEQ ID NO: 1, and/or a second polypeptide represented by one amino acid sequence selected from the group consisting of SEQ ID NO: 2 to SEQ ID NO: 5.

### [TECHNICAL SOLUTION]

Each description and embodiment disclosed in the present application can also be applied to each other description and embodiment. In other words, all combinations of the various elements disclosed in the present application belong to the scope of the present application. In addition, the scope of the present application cannot be considered limited by the specific description described below. Furthermore, those skilled in the art can recognize or confirm numerous equivalents to the specific aspect of the present application disclosed in the present application using only a common experiment. In addition, these equivalents are intended to be included in the present application.

In order to achieve the purposes of the present invention as above, the present invention provides an amorphous peptide tag in which the major amino acids are composed of charged and hydrophobic amino acids.

The charged amino acids of the amorphous peptide tag forming a self-assembly, in which major amino acids are composed of charged and hydrophobic amino acids may be selected from positively charged amino acids of Lysine (Lys, K), Arginine (Arg, R) and Histidine (His, H), and negatively charged amino acids of Aspartate (Asp, D) and Glutamate (Glu, E), and the hydrophobic amino acids may be selected from Alanine (Ala, A), Valine (Val, V), Leucine (Leu, L), Isoleucine (Ile, I), Proline (Pro, P), Phenylalanine (Phe, F), Methionine (Met, M) and Tryptophan (Trp, W), and it is characterized in that the content of the charged and hydrophobic amino acids in the alpha-helix tag excluding a hinge region or link site is 60% or more, 65% or more, 70% or more, preferably, 75% or more.

The amorphous peptide tag may be any one selected from the group consisting of PSIAEH tag, PSIAHEK tag, PSIHEKIHEK tag and PSIA tag, and may be a peptide capable of nucleation or formation of multimers such as dimers, and the like.

The amorphous peptide tag may comprise or consist of an amino acid sequence selected from the group consisting of SEQ ID NO: 2 to SEQ ID NO: 5.

The term used in the present description, " amorphous peptide tag" may mean a peptide comprising a disordered region that does not have a fixed conformation under normal condition, such as a secondary or tertiary structure of an alpha-helix or beta-helix, and the term "amorphous peptide tag" may be interchangeably used with "unstructured peptide tag" or "second polypeptide".

In addition, the present invention provides the amorphous peptide tag or a fusion protein in which the amorphous peptide tag and a target protein are fused, a polynucleotide encoding thereof, a vector expressing the same, and a host cell transformed with the expression vector.

Furthermore, the present invention provides a method for purifying a target protein comprising culturing a cell comprising a polynucleotide encoding the amorphous peptide tag or a recombinant fusion protein in which the peptide tag and a target protein are fused in a medium.

In addition, the present invention provides a composition for purifying a protein comprising the amorphous peptide tag, or comprising a fusion protein comprising the amorphous peptide tag and a target protein.

In addition, the present invention provides a fusion polypeptide comprising a first polypeptide comprising the amino acid sequence of SEQ ID NO: 1 and a second polypeptide having amorphous characteristics in which the major amino acids are composed of charged and hydrophobic amino acids.

The first polypeptide may be a purification tag. Specifically, the first polypeptide may be a His tag capable of binding to Ni²⁺ or Co²⁺, a HAT tag that is an original form thereof, or a modified HQ tag (or HQ₆ tag) of the His tag, but not limited thereto. The His tag may comprise the amino acid sequence of SEQ ID NO: 1, and may be represented by, or consist of the amino acid sequence of SEQ ID NO: 1.

The second polypeptide may be a motif, domain or peptide capable of nucleation or formation of multimers such as dimers and the like. Specifically, the second polypeptide may be any one selected from the group consisting of PSIAEH, PSIAHEK, PSIHEKIHEK and PSIA peptides, but not limited thereto.

The second polypeptide may comprise one amino acid sequence selected from the group consisting of SEQ ID NO: 2 to SEQ ID NO: 5 or consist of the same. The PSIAEH, PSIAHEK, PSIHEKIHEK and PSIA peptides may be represented by, or consist of an amino acid of SEQ ID NO: 2, SEQ ID NO: 3, SEQ ID NO: 4 and SEQ ID NO: 5, respectively.

The first polypeptide and the second polypeptide may be sequentially linked.

In addition, the present invention provides a fused recombinant fusion protein comprising a fusion polypeptide, which comprises the first polypeptide and the second polypeptide, and a target protein.

The fusion polypeptide which comprises the first polypeptide and the second polypeptide may be inserted into not only at the C-terminus of the target protein, but also at the N-terminus or anywhere within the protein as long as it does not substantially affect the functionality of the protein. Herein, that it does not substantially affect the functionality of the protein means that the activity of the protein before fusing the fusion polypeptide is maintained by 80% or more, preferably, 95% or more.

In the present invention, "recombinant protein" means a polymer of amino acids (peptides, oligopeptides, polypeptides or proteins) comprising at least 2 parts in which each part comprises a separate function. At least one first part of the recombinant protein comprises at least one fusion polypeptide comprising the first polypeptide and the second polypeptide of the present invention, and at least one second part of the recombinant protein comprises at least one target protein (or peptide).

In the present invention, "target protein" refers to any protein targeted for production or purification, including peptides, and may be interchangeably used with the term "targeted protein" in the present description.

As the target protein, green fluorescent protein (GFP), granulocyte macrophage colony stimulating factor (CSF), interferon alpha 2 (INF-α2) and TEV protease, but not limited thereto. The term, "target protein" refers to a protein to be produced by the biotechnological method according to the present invention, and is not particularly limited to any one, and preferably, includes proteins that can be used for medical, industrial, diagnostic, and experimental uses, and the like.

The fusion protein may further a cleavage site of protease, a hinge region of an immunoglobulin, the target protein or all of them between the fusion polypeptide, comprising the first polypeptide and the second polypeptide, and the target protein.

The hinge region may comprise the amino acid sequence of SEQ ID NO: 20, or consist of the same.

The fusion protein may comprise a cleavage site generally used for protein purification such as a TEV protease cleavage site or SNAC tag that can be cleaved by Ni²⁺, or the like between the fusion polypeptide and target protein to remove the fusion polypeptide comprising the first polypeptide and the second polypeptide in the recombinant fusion protein.

In addition, the present invention provides a fusion polypeptide comprising a first polypeptide and a second polypeptide, or a polynucleotide encoding a fusion protein comprising the fusion polypeptide and a target protein.

The polynucleotide comprises each polynucleotide encoding the first polypeptide and the second polypeptide. The polynucleotide encoding the first polypeptide may comprise the nucleotide sequence of SEQ ID NO: 21 or consist of the same, and the polynucleotide encoding the second polypeptide may comprise one nucleotide sequence selected from the nucleotide sequences of SEQ ID NO: 22 to SEQ ID NO: 25, or consist of the same.

The nucleotide sequences in the sequence listing described in the present description are described in the direction from the 5' end to the 3' end, even if there is no separate indication.

Furthermore, the present invention provides a recombinant expression vector comprising a fusion polypeptide comprising a first polypeptide and a second polypeptide, or a polynucleotide encoding a fusion protein comprising the fusion polypeptide and a target protein.

In the present invention, recombinant may be interchangeably used with genetic manipulation, and refers to producing a gene in a type that does not exist in nature by using a molecular cloning technique such as modifying, cutting, linking genes, and the like.

In the present invention, expression means that proteins or nucleic acids are generated in a cell.

In the present invention, recombinant expression vector is a vector capable of expressing a targeted protein or nucleic acid (RNA) in a suitable host cell, and refers to a gene construct comprising essential regulatory factors operably linked to express a polynucleotide (gene) insert.

In the present description, "operably linked" means that a nucleic acid expression regulatory sequence and a nucleic acid sequence encoding a targeted protein or RNA are under functional linkage to perform a general function, and that they are linked so that a gene can be expressed by the expression control sequence. The expression control sequence means a DNA sequence that controls expression of a polynucleotide sequence operably linked in a specific host cell. This control sequence comprises a promoter for conducting transcription, any operator sequence for controlling transcription, a sequence encoding an appropriate mRNA ribosome binding site, a sequence controlling transcription and translation, a start codon, a stop codon, a polyadenylated signal and an enhancer and the like.

The type of the recombinant expression vector of the present invention is not particularly limited as long as it is a vector commonly used in the cloning filed, and the examples include plasmid vectors, cosmid vectors, bacteriophage vectors and virus vectors and the like, but not limited thereto. The plasmids include E. coli-derived plasmids (pBR322, pBR325, pUC118 and pUC119, pET-22(+)), Bacillus subtilis-derived plasmids (pUB110 and pTP5) and yeast-derived plasmids (pPICZ, YEp13, YEp24 and YCp50), and the like, and as the viruses, animal viruses such as retrovirus, adenovirus, or vaccinia virus, insect viruses such as baculovirus, and the like may be used, and preferably, pET-28a vector may be used.

In addition, the present invention provides a host cell transformed with an expression vector comprising a polynucleotide encoding a fusion polypeptide comprising a first polypeptide and a second polypeptide, or a fusion protein comprising the fusion polypeptide and a target protein.

The type of the host cell according to the present invention is not particularly limited as long as it is a cell that can be used for expressing the polynucleotide comprised in the recombinant expression vector of the present invention. The cell (host cell) transformed with the recombinant expression vector according to the present invention may be a prokaryote (for example, *E. coli),* an eukaryote (for example, yeast or other fungus), a plant cell (for example, tobacco or tomato plant cell), an animal cell (for example, human cell, monkey cell, hamster cell, rat cell, mouse cell), an insect cell, or a hybridoma derived therefrom, and preferably, may be *E. coli,* but not limited thereto.

The recombinant expression vector according to the present invention may transform it by being introduced inside a cell for producing an antibody or fragment thereof by a known method in the art, for example, not limited thereto, transient transfection, microinjection, transduction, cell fusion, calcium phosphate precipitation, liposome-mediated transfection, DEAE dextran-mediated transfection, polybrene-mediated transfection, electroporation, gene gun and a known method for flowing a nucleic acid into a cell. The cell transformed with the recombinant expression vector according to the present invention may overexpress or mass-produce the fusion protein according to the present invention.

Furthermore, the present invention provides a method for purifying a target protein, comprising culturing a host cell transformed with an expression vector comprising a polynucleotide encoding a fusion polypeptide comprising a first polypeptide and a second polypeptide, or a fusion protein comprising the fusion polypeptide and a target protein in a medium.

The contents of the fusion polypeptide or target protein are as described above.

The method for purifying a target protein may further comprise extracting the fusion protein from the host cell, after the culturing.

The buffer solution used in the extracting the fusion protein may be a buffer solution generally (commonly) used for extracting proteins such as HEPES, Tris, or phosphate, or the like, and preferably, it may be HEPES buffer solution, but not limited thereto.

In the extracting the fusion protein, a surfactant such as Trion X-100 may be added to increase cell lysis efficiency, and a protease inhibitor cocktail in which various protease inhibitors are mixed may be added to inhibit proteolysis.

The method for purifying a target protein may further comprise inducing formation of a self-assembly of the fusion protein obtained from a host cell transformed with an expression vector comprising a polynucleotide encoding a fusion polypeptide comprising a first polypeptide and a second polypeptide, or a fusion protein comprising the fusion polypeptide and a target protein.

The inducing formation of a self-assembly may further comprise treating under specific conditions for promoting formation of a self-assembly. The specific conditions for inducing formation of a self-assembly may be physical·chemical conditions such as the kind of inducer, concentration of the inducer, the type of salt, the concentration of salt, pH condition, the type of buffer solution, temperature, reaction time and the like.

The inducing formation of a self-assembly may comprise adding an inducer for promoting formation of a self-assembly. According to adding the inducer, induction of formation of a macromolecular self-assembly may be promoted.

The inducer may be a cationic substance, and specifically, may be any one or more selected from the group consisting of cations such as Ni²⁺, Co²⁺, Zn²⁺, Cu²⁺, Ag²⁺, Fe²⁺ and Ba²⁺, and the type of the appropriate inducer for inducing formation of a self-assembly may be different depending on the combination of the first polypeptide and the second polypeptide.

The inducer may be 0.1 to 2.0 mM, 0.1 to 1.5 mM, 0.1 to 1.0 mM, 0.1 to 0.7 mM 0.3 to 2.0 mM, 0.3 to 1.5 mM, 0.3 to 1.0 mM, 0.3 to 0.7 mM, 0.4 to 2.0 mM, 0.4 to 1.5 mM, 0.4 to 1.0 mM, 0.4 to 0.7 mM, 0.4 to 0.6 mM, or 0.5 mM, but not limited thereto.

The inducing formation of a self-assembly may comprise adding a salt. The salt may be a salt generally used for protein precipitation such as ammonium sulfate or sodium chloride, or the like, and preferably, may be ammonium sulfate, but not limited thereto.

The ammonium sulfate may be 5 to 30% (w/v), 5 to 27% (w/v), 5 to 25% (w/v), 5 to 23% (w/v), 7 to 30% (w/v), 7 to 27% (w/v), 7 to 25% (w/v), 7 to 23% (w/v), 10 to 30% (w/v), 10 to 27% (w/v), 10 to 25% (w/v), 10 to 23% (w/v), 12 to 30% (w/v), 12 to 27% (w/v), 12 to 25% (w/v), 12 to 23% (w/v), or 12 to 22% (w/v), but not limited thereto.

The buffer solution used in the inducing formation of a self-assembly may be a buffer solution generally used for extracting proteins such as HEPES, Tris, or phosphate, or the like, and preferably, it may be HEPES buffer solution, but not limited thereto.

The inducing formation of a self-assembly may be performed at pH 6 to pH 10, and may be performed at pH 7 to pH 10, but not limited thereto.

The inducing formation of a self-assembly may be performed immediately after treating the inducer to for 10 minutes, 10 minutes or more, 15 minutes or more, 20 minutes or more, 10 minutes to 120 minutes, 15 minutes to 120 minutes, 20 minutes to 120 minutes, but not limited thereto.

The method for purifying a target protein may further comprise selectively separate the formed self-assembly.

The separating may be performed by a centrifugation method or filtration method.

The fusion protein comprising a fusion polypeptide which comprises a first polypeptide and a second polypeptide and a target protein forms a macromolecular self-assembly to increase the size and density, so the centrifugation method that can precipitate it using a density difference or the filtration method that can filter molecules bigger than a certain size may be used as a separation method.

In order to minimize contamination of non-specific protein that may remain after the separating of the centrifugation method or filtration method, washing recombinant protein fractions using a buffer solution may be further comprised.

Furthermore, the present invention is a method for reversibly converting a recombinant protein purified by inducing formation of a macromolecular self-assembly through specific inducer treatment and/or condition treatment into monomers, and may further comprise treating a chelator such as EDTA or EGTA that can strongly bind to a specific cationic inducer.

The method for purifying a target protein may further comprise cleaving the TEV protease cleavage site or SNAC tag comprised in the fusion protein, and the cleaving may be performed by TEV protease or Ni²⁺ treatment.

In addition, the present invention provides a composition for purifying a protein comprising an amorphous peptide tag having amorphous characteristics in that major amino acids are composed of charged and hydrophobic amino acids.

The polypeptide tag may be any one selected from the group consisting of PSIAEH tag, PSIAHEK tag, PSIHEKIHEK tag and PSIA tag, and may be a peptide capable of nucleation or formation of multimers such as dimers and the like.

The amorphous polypeptide tag may comprise one amino acid sequence selected from the group consisting of SEQ ID NO: 2 to SEQ ID NO: 5, or consist of the same.

Furthermore, the present invention provides a self-assembly comprising an amorphous peptide tag having amorphous characteristics in which the major amino acids are composed of charged and hydrophobic amino acids.

The amorphous polypeptide tag may be any one selected from the group consisting of PSIAEH tag, PSIAHEK tag, PSIHEKIHEK tag and PSIA tag, and may be a peptide capable of nucleation or formation of multimers such as dimers and the like.

The polypeptide tag may comprise one amino acid sequence selected from the group consisting of SEQ ID NO: 2 to SEQ ID NO: 5, or consist of the same.

### [ADVANTAGEOUS EFFECTS]

The present invention relates to an amorphous polypeptide in which the major amino acids are composed of charged and hydrophobic amino acids, a fusion protein in which the tag is fused with a target protein, and a method for purifying a target protein using the same, and the peptide alone or fusion protein induces formation of macromolecular self-assemblies in a specific inducer or treatment condition dependent manner, thereby making it possible to separate and purify a recombinant protein only by a very simple method such as centrifugation or filtration without the need for a purification column filled with expensive beads or resins mainly used in conventional affinity chromatography and an expensive device or equipment operating thereof. Furthermore, the present invention has advantages of a very simple and easy treatment process, a very short time required for purification, and being able to purify recombinant proteins with high efficiency and high purity, since macromolecular self-assembly formation is induced through salt treatment and specific inducer or condition treatment to a protein extraction solution. In addition, the present invention can easily convert a purified recombinant protein into water-soluble monomer through chelate treatment, and can easily remove these tags by inducing reformation of self-assemblies after cutting tags fused to a target protein if needed.

### [BRIEF DESCRIPTION OF THE DRAWINGS]

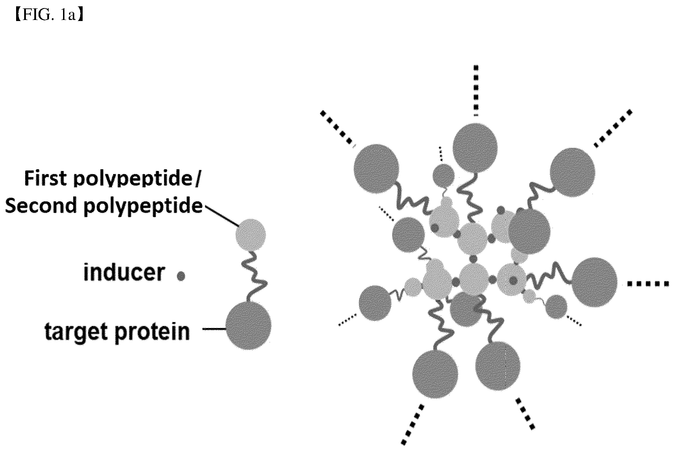

FIG. 1a is a diagram showing a concept of inducing formation of a macromolecular self-assembly by a specific inducer acting on a fused purification tag and a self-assembly tag.

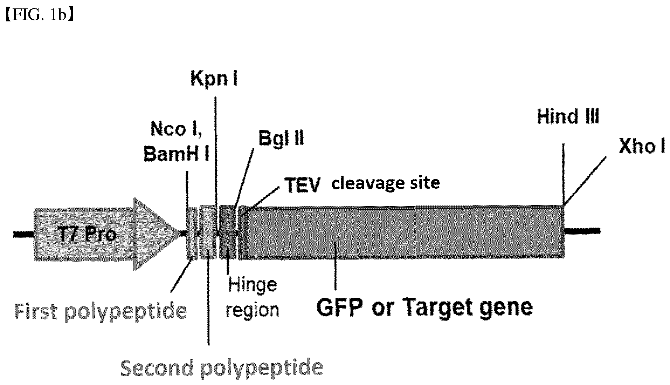

FIG. 1b is a schematic diagram showing the configuration of a vector for expressing a target protein comprising a gene region encoding a His₆ tag and a self-assembly tag, and comprising a gene region encoding a target protein.

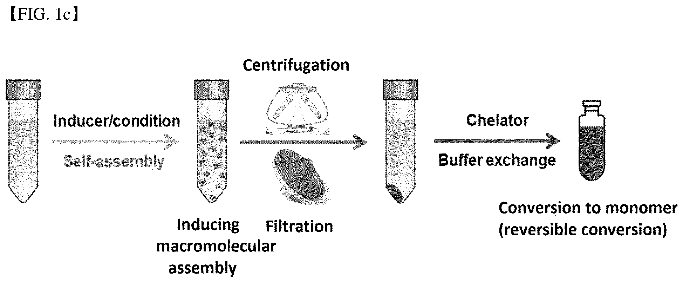

FIG. 1c is a diagram showing a method for selectively purifying a recombinant protein by a method such as centrifugation or filtration or the like after inducing formation of a macromolecular self-assembly through a specific inducer or specific condition treatment.

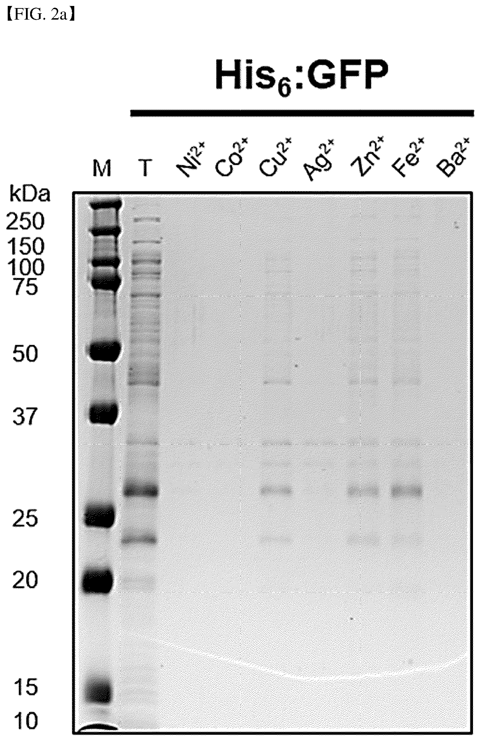

FIG. 2a is a diagram confirming whether formation of a macromolecular self-assembly is induced when various cations are treated, by fusing a His tag alone as a purification tag to a target protein, GFP.

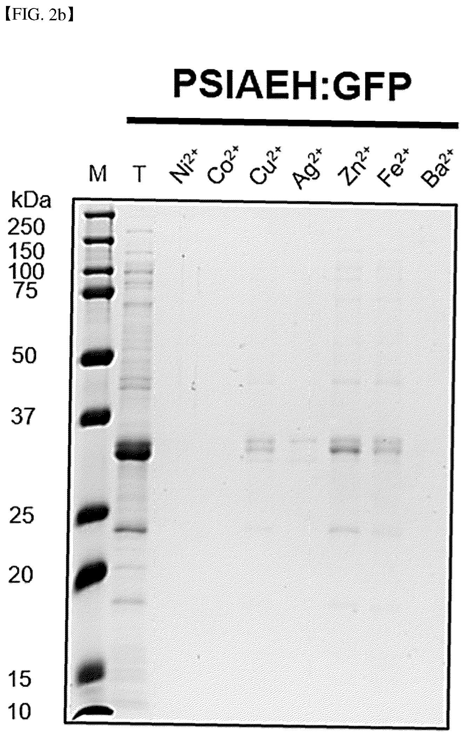

FIG. 2b is a diagram confirming whether formation of a macromolecular self-assembly is induced when various cations are treated, by fusing a PSIAEH peptide tag alone, which is artificially designed(de novo designed) to enable nucleation or formation of multimers such as dimers, as purification tags to GFP.

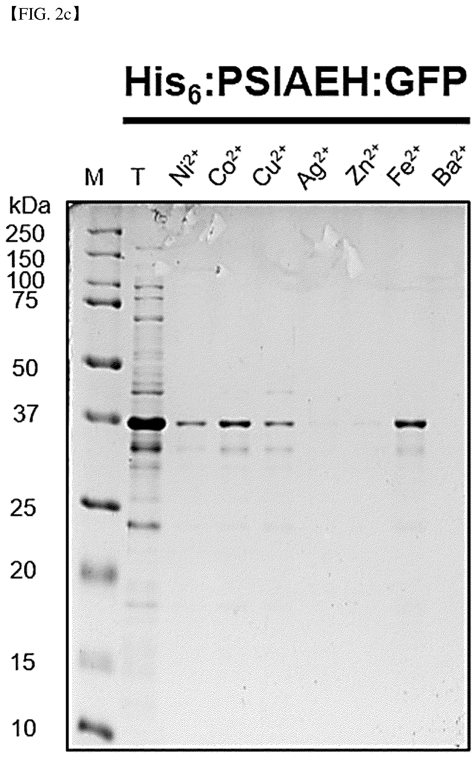

FIG. 2c is a diagram confirming whether formation of a macromolecular self-assembly is induced when various cations are treated, by fusing a combination of a His tag and a PSIAEH peptide tag as purification tags to GFP.

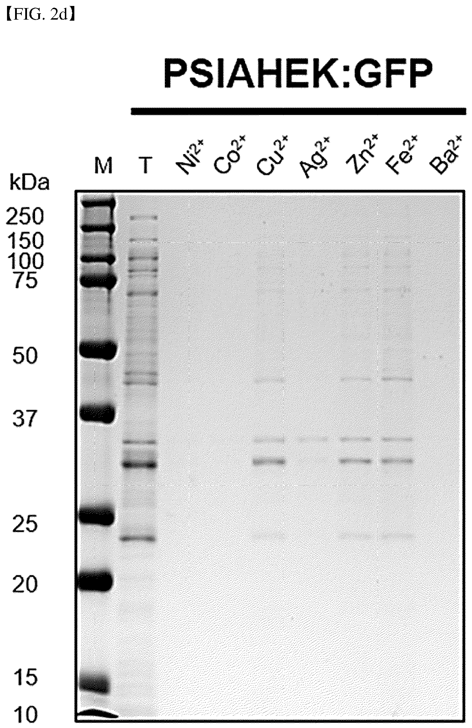

FIG. 2d is a diagram confirming whether formation of a macromolecular self-assembly is induced when various cations are treated, by fusing a PSIAHEK peptide tag alone, which is artificially designed(a de novo designed) to enable nucleation or formation of multimers such as dimers, as purification tags to GFP .

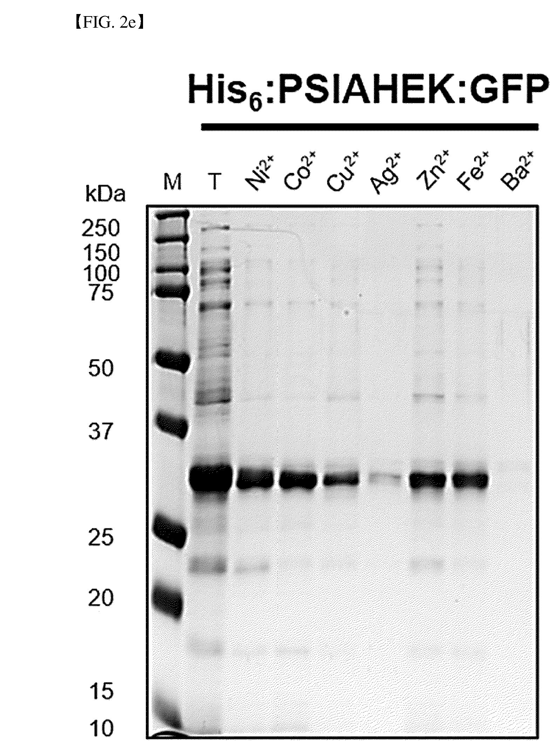

FIG. 2e is a diagram confirming whether formation of a macromolecular self-assembly is induced when various cations are treated, by fusing a combination of a His tag and a PSIAHEK peptide tag as purification tags to GFP.

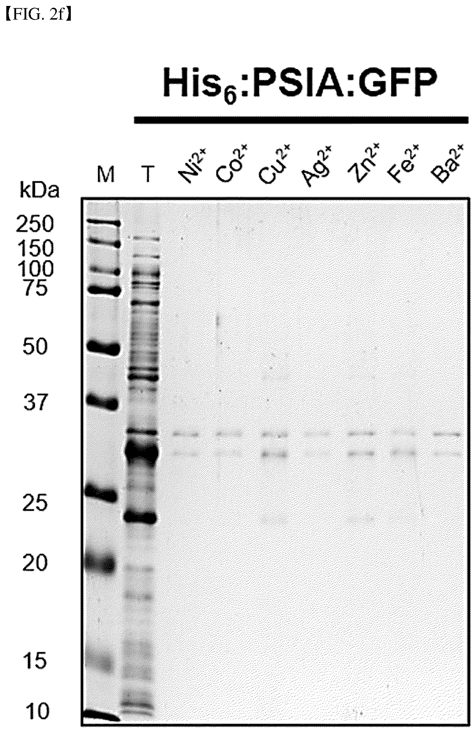

FIG. 2f is a diagram confirming whether formation of a macromolecular self-assembly is induced when various cations are treated, by fusing a combination of a His tag and a PSIA peptide tag as purification tags to GFP.

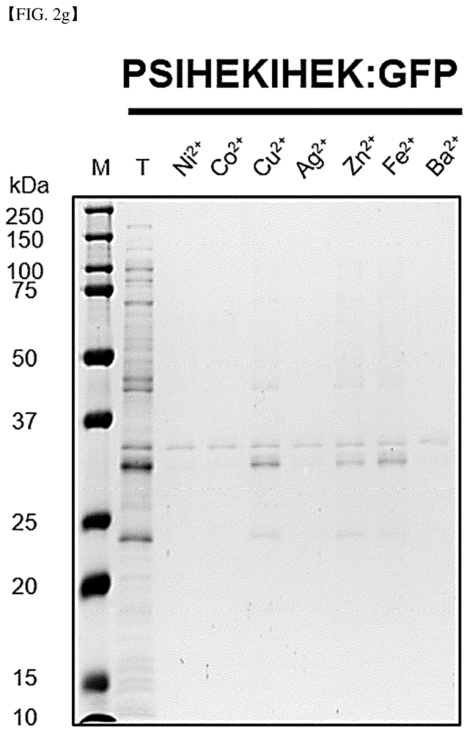

FIG. 2g is a diagram confirming whether formation of a macromolecular self-assembly is induced when various cations are treated, by fusing a PSIHEKIHEK peptide tag alone, which is artificially designed(a de novo designed) to enable nucleation or formation of multimers such as dimers, as purification tags to GFP.

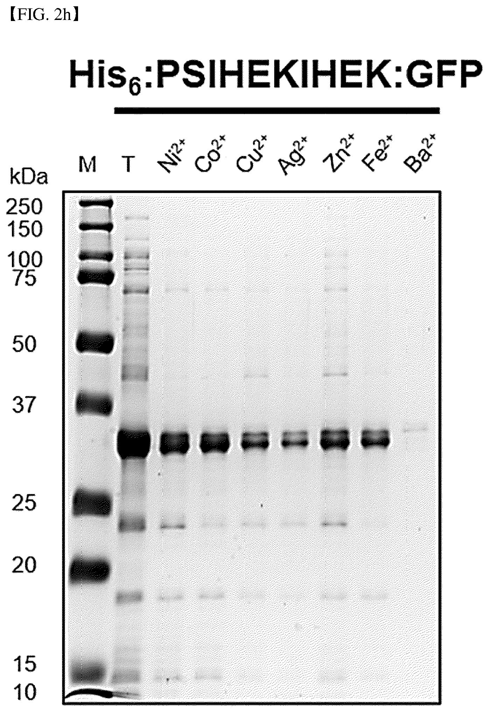

FIG. 2h is a diagram confirming whether formation of a macromolecular self-assembly is induced when various cations are treated, by fusing a combination of a His tag and a PSIHEKIHEK peptide tag as purification tags to GFP.

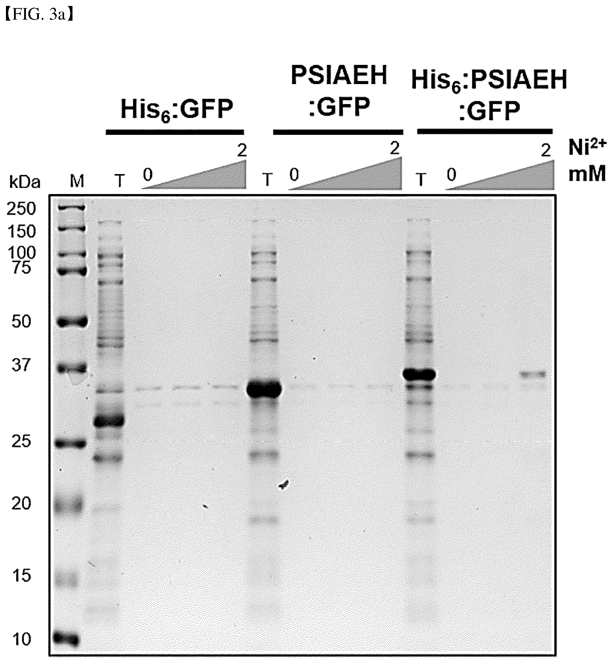

FIG. 3a is a diagram confirming whether formation of a macromolecular self-assembly is induced when cation Ni²⁺ is treated at various concentrations, by fusing a His tag or PSIAEH peptide tag alone or fusing both of these two tags as purification tags to GFP which is one of model proteins as a target protein.

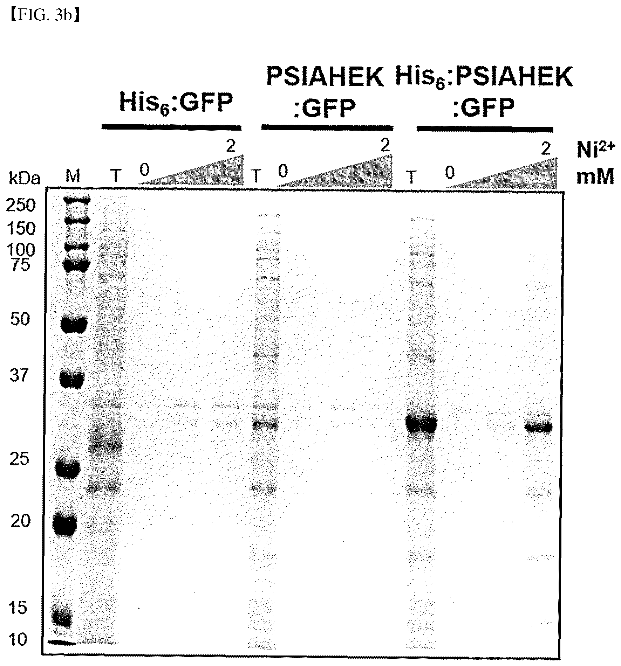

FIG. 3b is a diagram confirming whether formation of a macromolecular self-assembly is induced when cation Ni²⁺ is treated at various concentrations, by fusing a His tag or PSIAHEK peptide tag alone or fusing both of these two tags as purification tags to GFP.

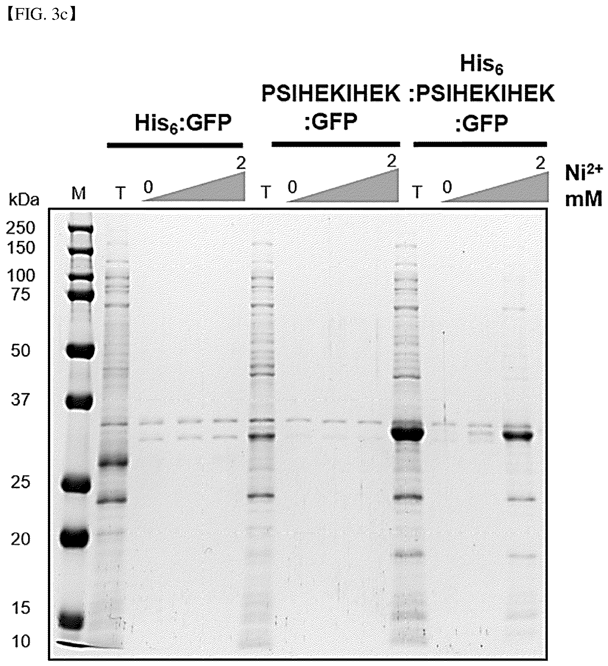

FIG. 3c is a diagram confirming whether formation of a macromolecular self-assembly is induced when cation Ni²⁺ is treated at various concentrations, by fusing a His tag or PSIHEKIHEK peptide tag alone or fusing both of these two tags as purification tags to GFP.

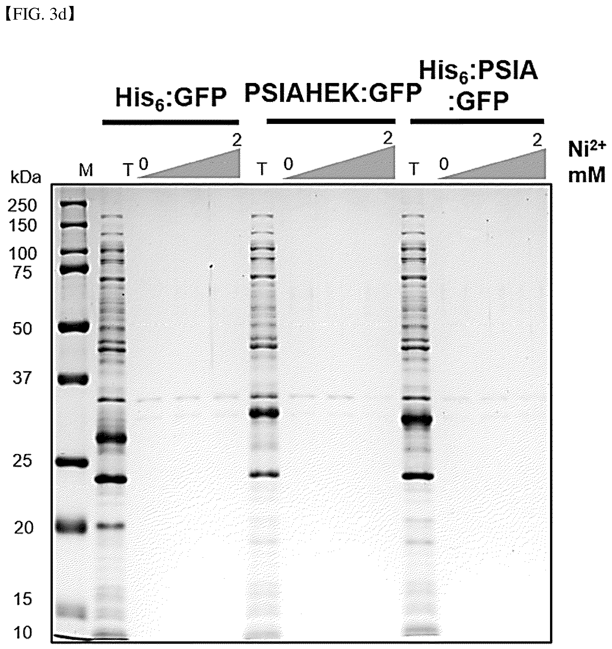

FIG. 3d is a diagram confirming whether formation of a macromolecular self-assembly is induced when cation Ni²⁺ is treated at various concentrations, by fusing a His tag or PSIAHEK peptide tag alone or fusing a combination of these two tags as purification tags to GFP.

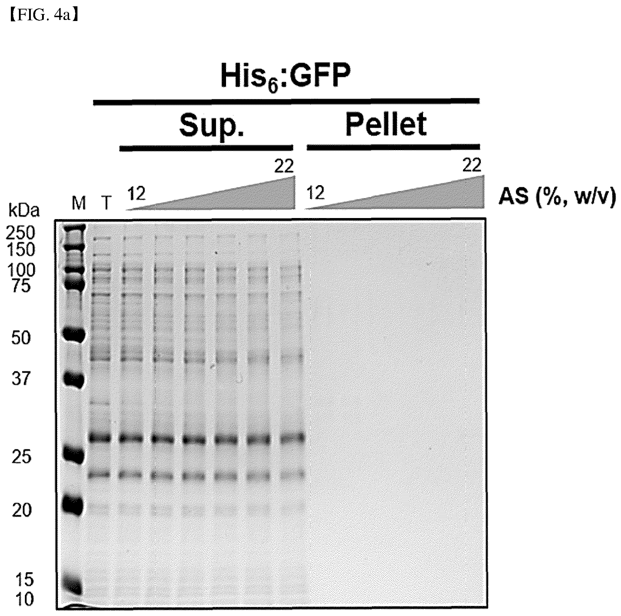

FIG. 4a is a diagram analyzing whether formation of a macromolecular self-assembly is promoted when cation Ni²⁺ selected as a specific inducer is treated under the condition where various concentrations of ammonium sulfate are present as salt, by fusing a His tag alone as a purification tag to GFP.

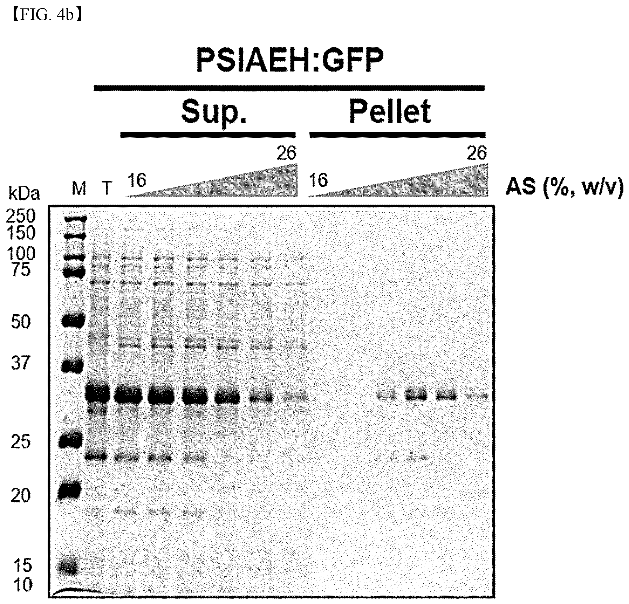

FIG. 4b is a diagram analyzing whether formation of a macromolecular self-assembly is promoted when cation Ni²⁺ selected as a specific inducer is treated under the condition where various concentrations of ammonium sulfate are present as salt, by fusing a PSIAEH peptide tag alone as a purification tag to GFP.

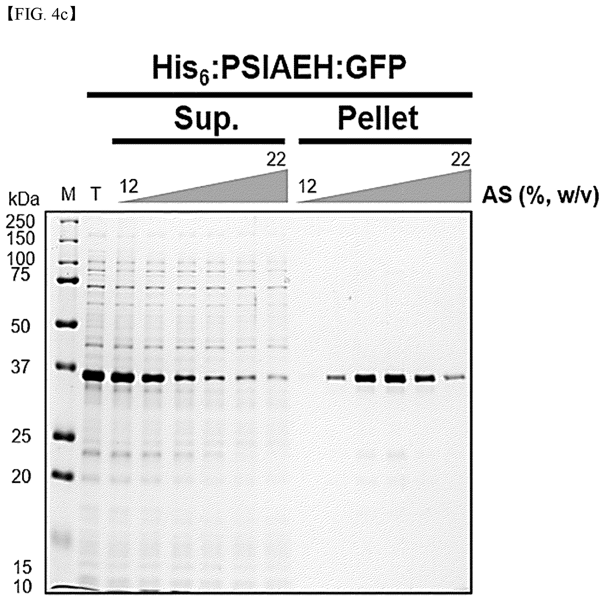

FIG. 4c is a diagram analyzing whether formation of a macromolecular self-assembly is promoted when cation Ni²⁺ selected as a specific inducer is treated under the condition where various concentrations of ammonium sulfate are present as salt, by fusing a combination of a His tag and a PSIAEH peptide tag as purification tags to GFP.

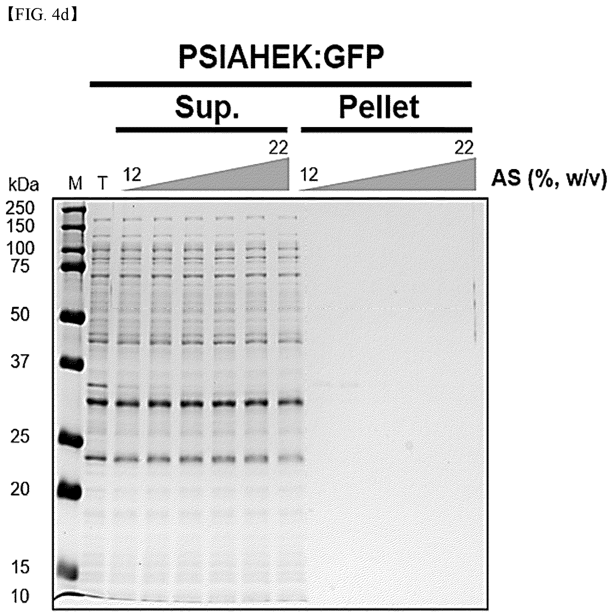

FIG. 4d is a diagram analyzing whether formation of a macromolecular self-assembly is promoted when cation Ni²⁺ selected as a specific inducer is treated under the condition where various concentrations of ammonium sulfate are present as salt, by fusing a PSIAHEK peptide tag alone as a purification tag to GFP.

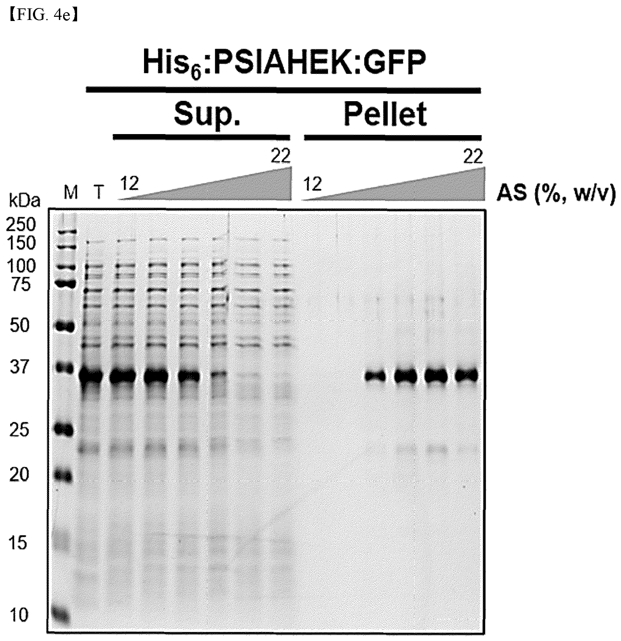

FIG. 4e is a diagram analyzing whether formation of a macromolecular self-assembly is promoted when cation Ni²⁺ selected as a specific inducer is treated under the condition where various concentrations of ammonium sulfate are present as salt, by fusing a combination of a His tag and a PSIAHEK peptide tag as purification tags to GFP.

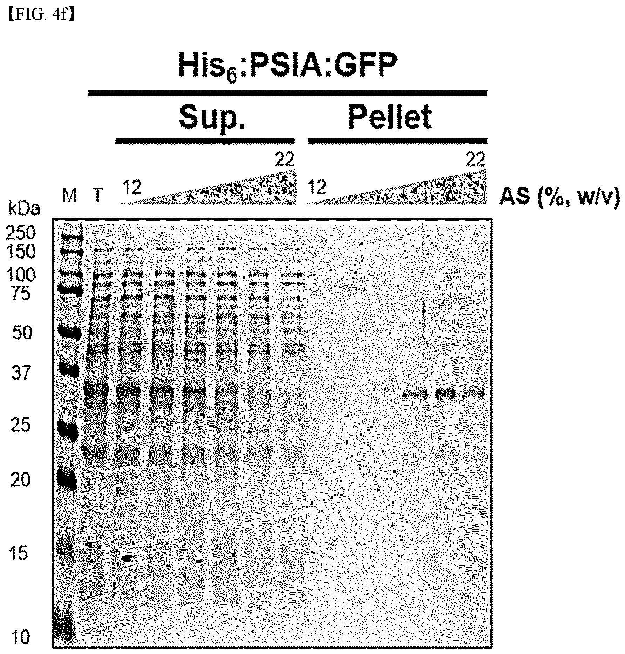

FIG. 4f is a diagram analyzing whether formation of a macromolecular self-assembly is promoted when cation Ni²⁺ selected as a specific inducer is treated under the condition where various concentrations of ammonium sulfate are present as salt, by fusing a combination of a His tag and a PSIA peptide tag as purification tags to GFP.

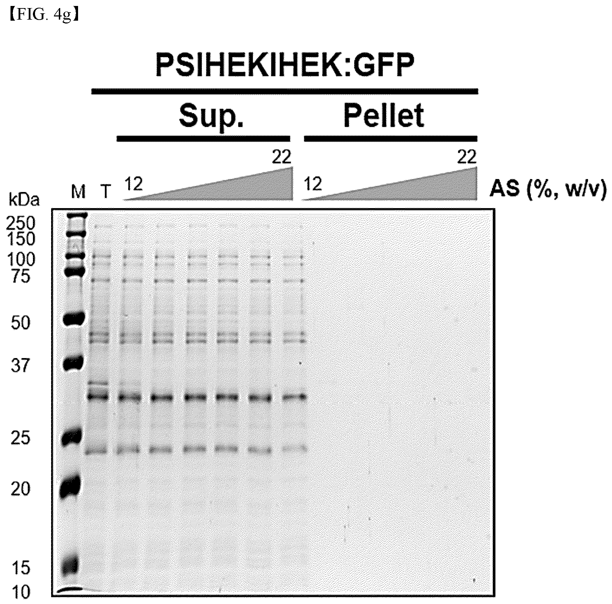

FIG. 4g is a diagram analyzing whether formation of a macromolecular self-assembly is promoted when cation Ni²⁺ selected as a specific inducer is treated under the condition where various concentrations of ammonium sulfate are present as salt, by fusing a PSIHEKIHEK peptide tag alone as a purification tag to GFP.

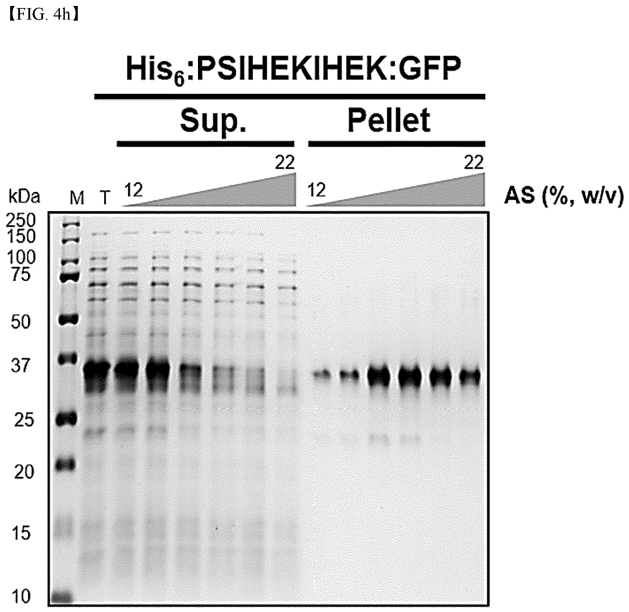

FIG. 4h is a diagram analyzing whether formation of a macromolecular self-assembly is promoted when cation Ni²⁺ selected as a specific inducer is treated under the condition where various concentrations of ammonium sulfate are present as salt, by fusing a combination of a His tag and a PSIHEKIHEK peptide tag as purification tags to GFP.

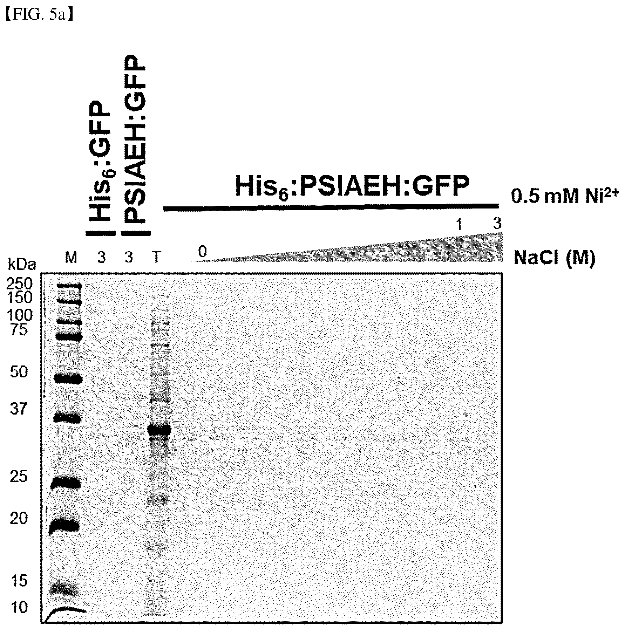

FIG. 5a is a diagram analyzing whether formation of a macromolecular self-assembly is promoted when cation Ni²⁺ selected as a specific inducer is treated under the condition where various concentrations of sodium chloride are present as salt, by fusing a combination of a His tag and a PSIAEH peptide tag as purification tags to GFP.

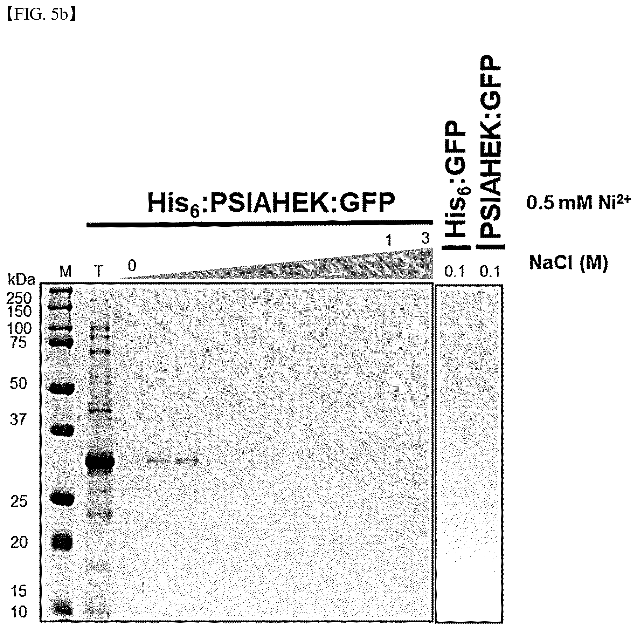

FIG. 5b is a diagram analyzing whether formation of a macromolecular self-assembly is promoted when cation Ni²⁺ selected as a specific inducer is treated under the condition where various concentrations of sodium chloride are present as salt, by fusing a combination of a His tag and a PSIAHEK peptide tag as purification tags to GFP.

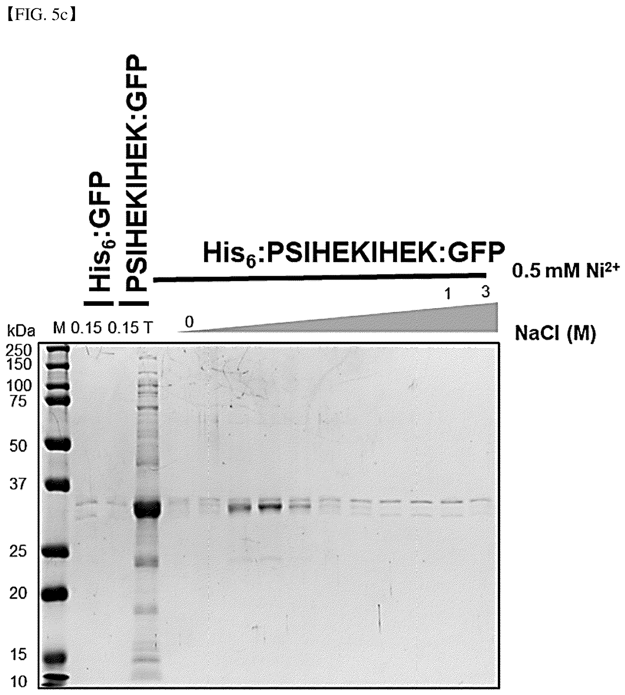

FIG. 5c is a diagram analyzing whether formation of a macromolecular self-assembly is promoted when cation Ni²⁺ selected as a specific inducer is treated under the condition where various concentrations of sodium chloride are present as salt, by fusing a combination of a His tag and a PSIHEKIHEK peptide tag as purification tags to GFP.

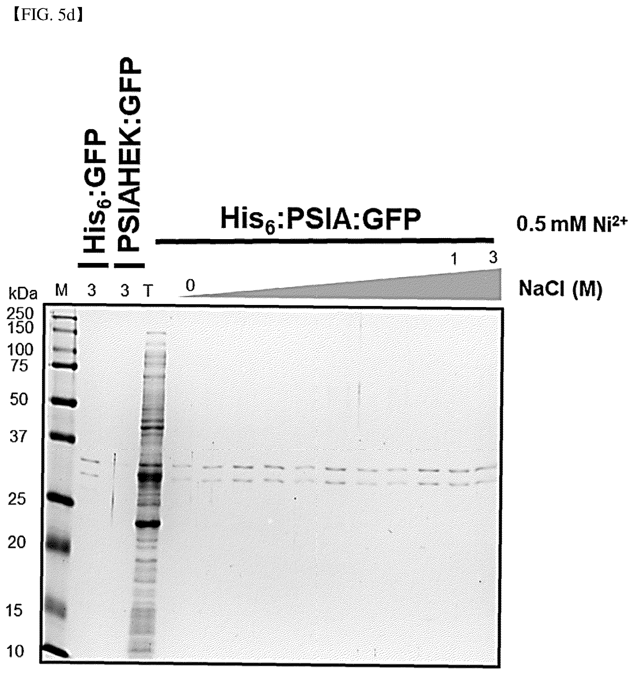

FIG. 5d is a diagram analyzing whether formation of a macromolecular self-assembly is promoted when cation Ni²⁺ selected as a specific inducer is treated under the condition where various concentrations of sodium chloride are present as salt, by fusing a combination of a His tag and a PSIA peptide tag as purification tags to GFP.

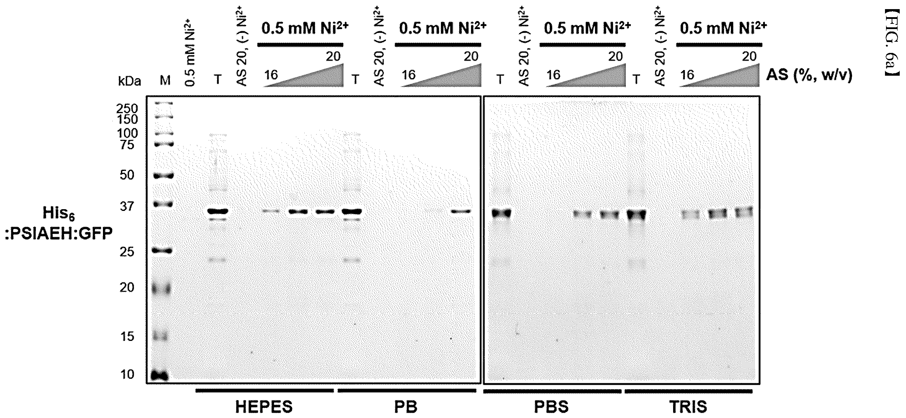

FIG. 6a is a diagram analyzing the degree of formation of a macromolecular self-assembly when ammonium sulfate is treated as salt and cation Ni²⁺ selected as a specific inducer is treated to various buffers by fusing a combination of a His tag and a PSIAEH peptide tag as purification tags to GFP.

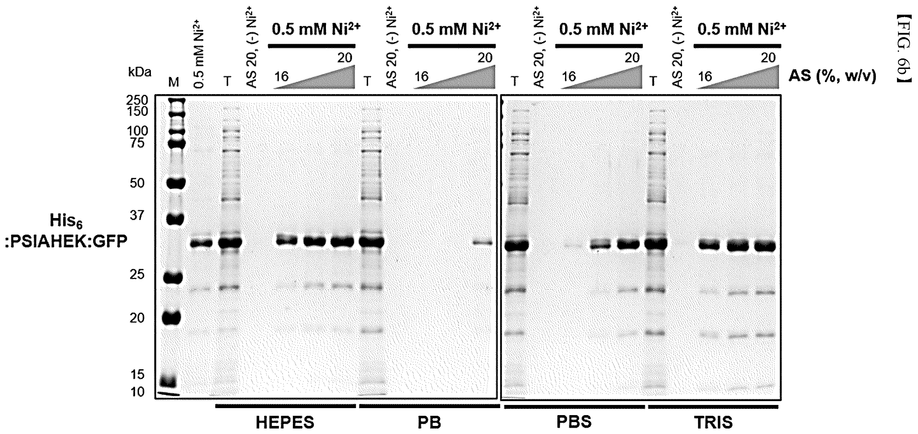

FIG. 6b is a diagram analyzing the degree of formation of a macromolecular self-assembly when ammonium sulfate is treated as salt and cation Ni²⁺ selected as a specific inducer is treated to various buffers by fusing a combination of a His tag and a PSIAHEK peptide tag as purification tags to GFP.

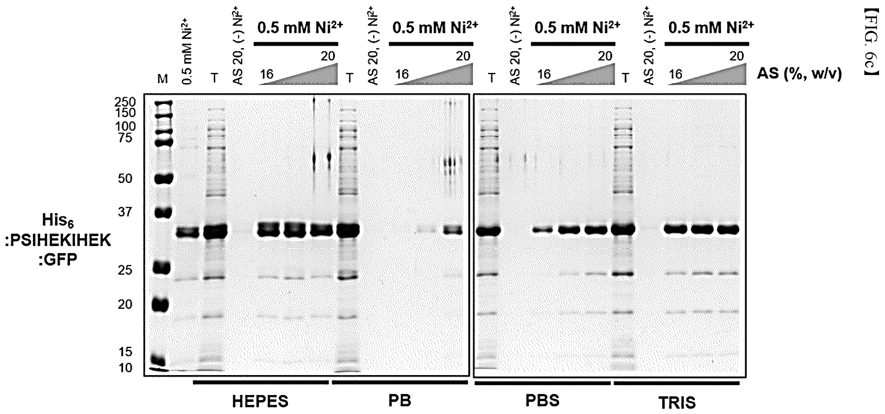

FIG. 6c is a diagram analyzing the degree of formation of a macromolecular self-assembly when ammonium sulfate is treated as salt and cation Ni²⁺ selected as a specific inducer is treated to various buffers by fusing a combination of a His tag and a PSIHEKIHEK peptide tag as purification tags to GFP.

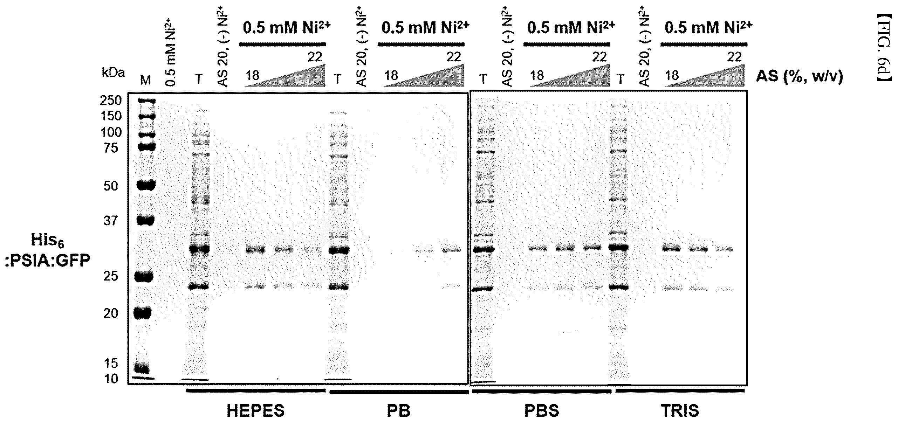

FIG. 6d is a diagram analyzing the degree of formation of a macromolecular self-assembly when ammonium sulfate is treated as salt and cation Ni²⁺ selected as a specific inducer is treated to various buffers by fusing a combination of a His tag and a PSIA peptide tag as purification tags to GFP.

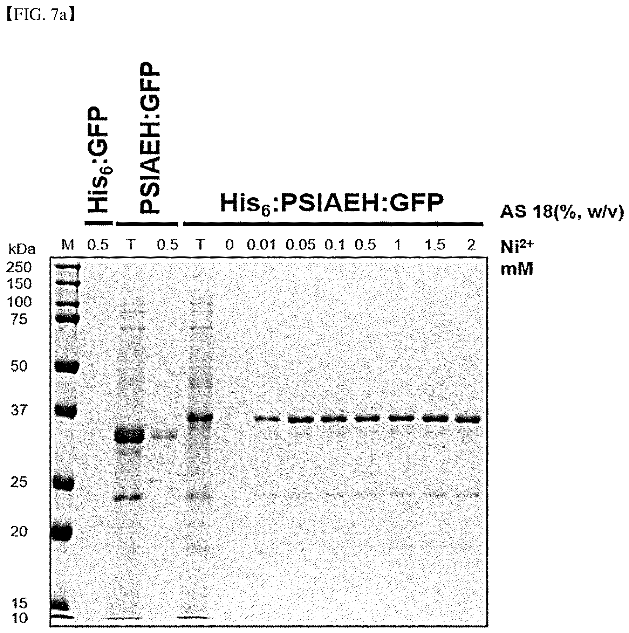

FIG. 7a is a diagram analyzing the degree of formation of a macromolecular self-assembly depending on the concentration of treatment of cation Ni²⁺ selected as a specific inducer under the condition where ammonium sulfate is present as salt, by fusing a combination of a His tag and a PSIAEH peptide tag as purification tags to GFP.

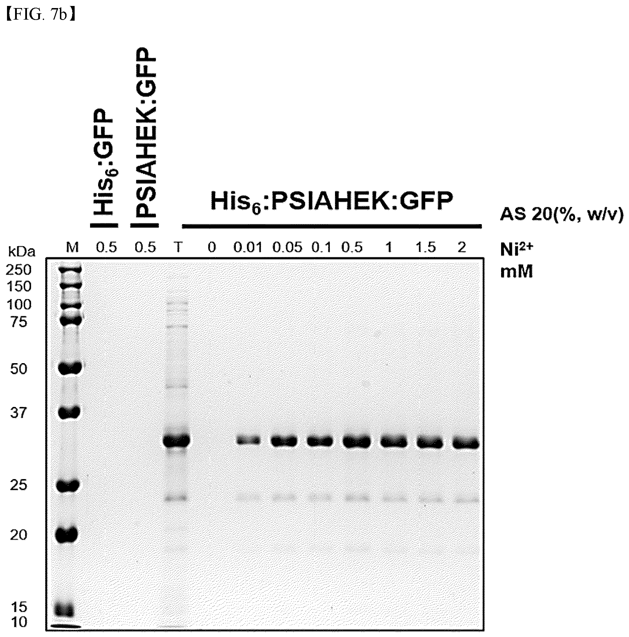

FIG. 7b is a diagram analyzing the degree of formation of a macromolecular self-assembly depending on the concentration of treatment of cation Ni²⁺ selected as a specific inducer under the condition where ammonium sulfate is present as salt, by fusing a combination of a His tag and a PSIAHEK peptide tag as purification tags to GFP.

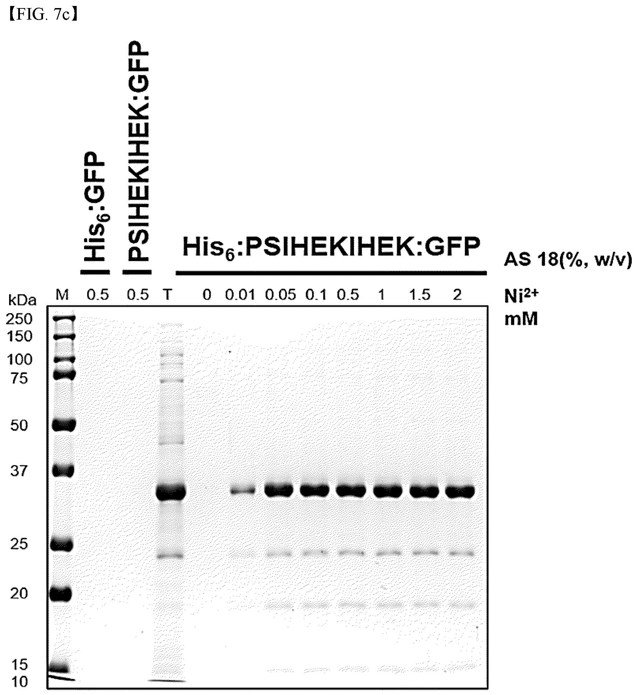

FIG. 7c is a diagram analyzing the degree of formation of a macromolecular self-assembly depending on the concentration of treatment of cation Ni²⁺ selected as a specific inducer under the condition where ammonium sulfate is present as salt, by fusing a combination of a His tag and a PSIHEKIHEK peptide tag as purification tags to GFP.

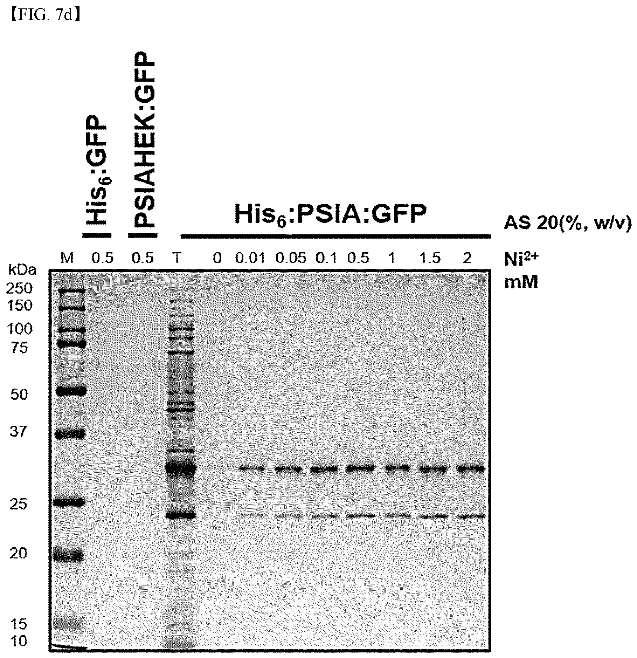

FIG. 7d is a diagram analyzing the degree of formation of a macromolecular self-assembly depending on the concentration of treatment of cation Ni²⁺ selected as a specific inducer under the condition where ammonium sulfate is present as salt, by fusing a combination of a His tag and a PSIA peptide tag as purification tags to GFP.

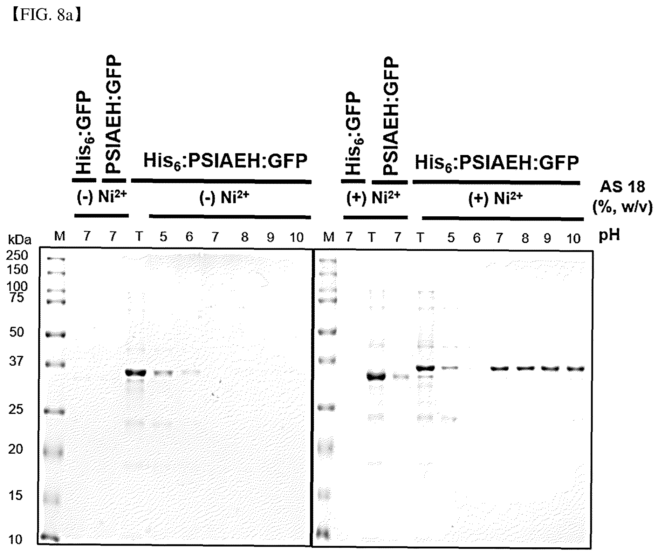

FIG. 8a is a diagram analyzing the degree of formation of a macromolecular self-assembly when ammonium sulfate is treated as salt and cation Ni²⁺ selected as a specific inducer is untreated or treated under various pH conditions of buffer, by fusing a combination of a His tag and a PSIAEH peptide tag as purification tags to GFP.

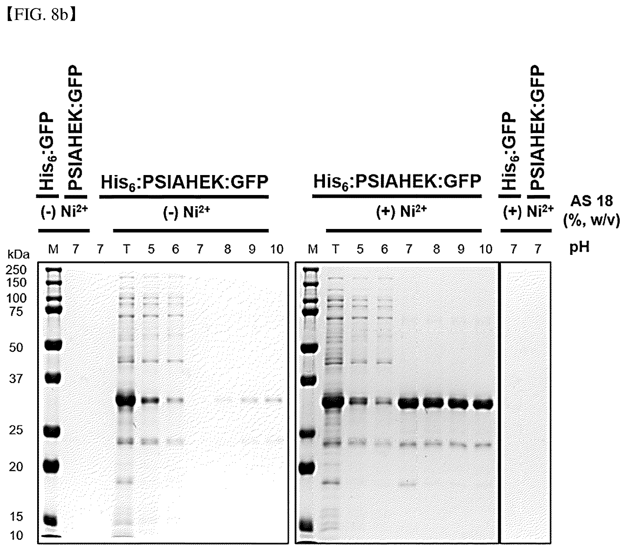

FIG. 8b is a diagram analyzing the degree of formation of a macromolecular self-assembly when ammonium sulfate is treated as salt and cation Ni²⁺ selected as a specific inducer is untreated or treated under various pH conditions of buffer, by fusing a combination of a His tag and a PSIAHEK peptide tag as purification tags to GFP.

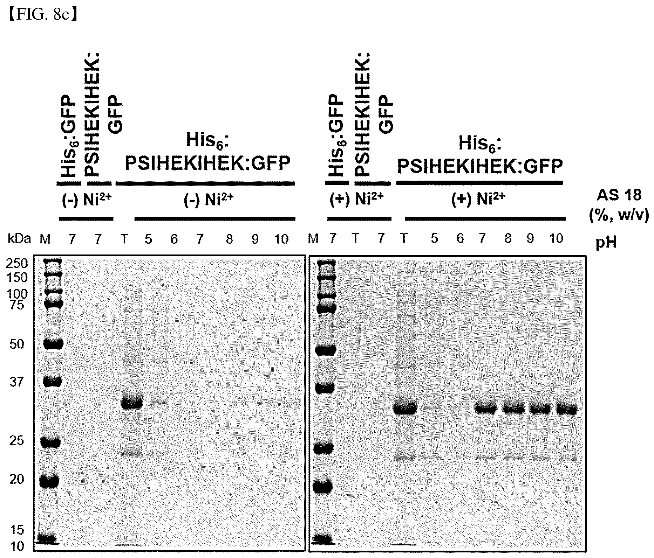

FIG. 8c is a diagram analyzing the degree of formation of a macromolecular self-assembly when ammonium sulfate is treated as salt and cation Ni²⁺ selected as a specific inducer is untreated or treated under various pH conditions of buffer, by fusing a combination of a His tag and a PSIHEKIHEK peptide tag as purification tags to GFP.

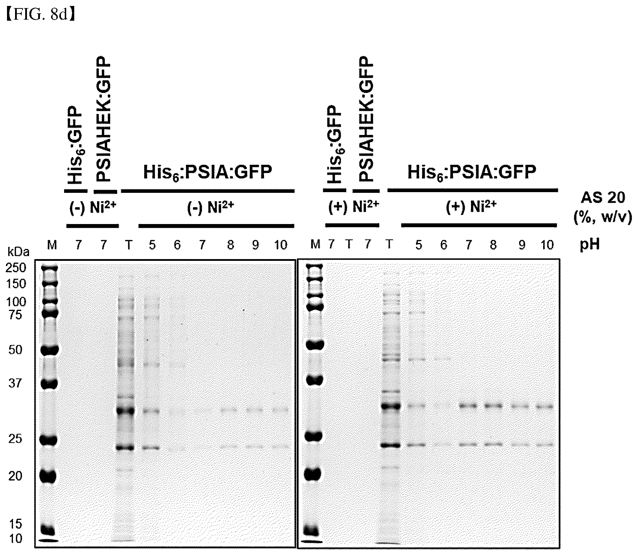

FIG. 8d is a diagram analyzing the degree of formation of a macromolecular self-assembly when ammonium sulfate is treated as salt and cation Ni²⁺ selected as a specific inducer is untreated or treated under various pH conditions of buffer, by fusing a combination of a His tag and a PSIA peptide tag as purification tags to GFP.

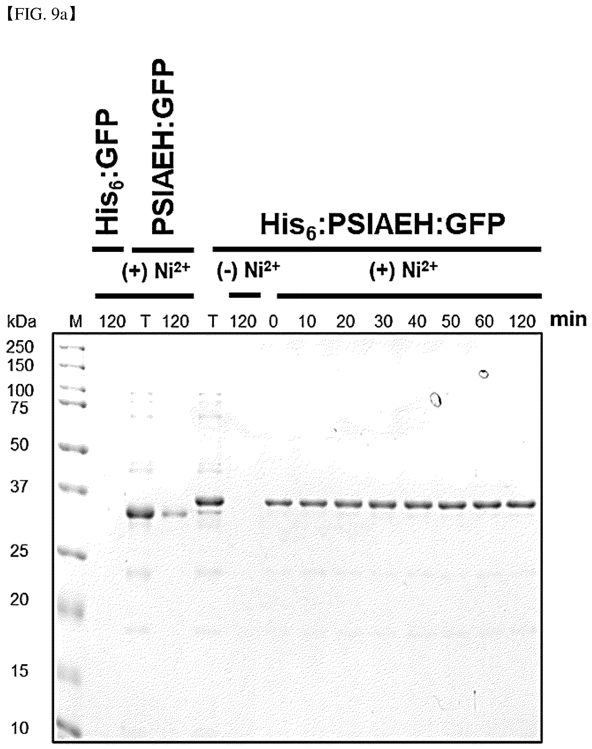

FIG. 9a is a diagram analyzing the degree of formation of a macromolecular self-assembly depending on the treatment time of cation Ni²⁺ selected as a specific inducer under the condition where ammonium sulfate is present as salt, by fusing a combination of a His tag and a PSIAEH peptide tag as purification tags to GFP.

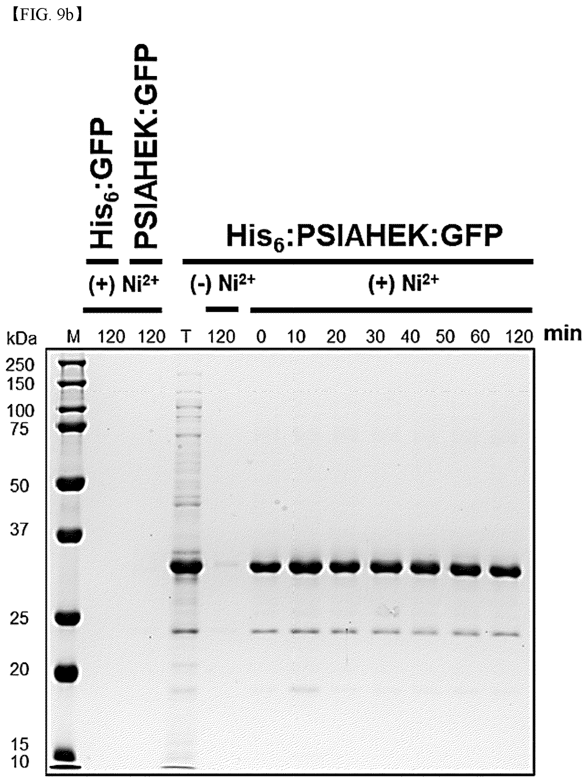

FIG. 9b is a diagram analyzing the degree of formation of a macromolecular self-assembly depending on the treatment time of cation Ni²⁺ selected as a specific inducer under the condition where ammonium sulfate is present as salt, by fusing a combination of a His tag and a PSIAHEK peptide tag as purification tags to GFP.

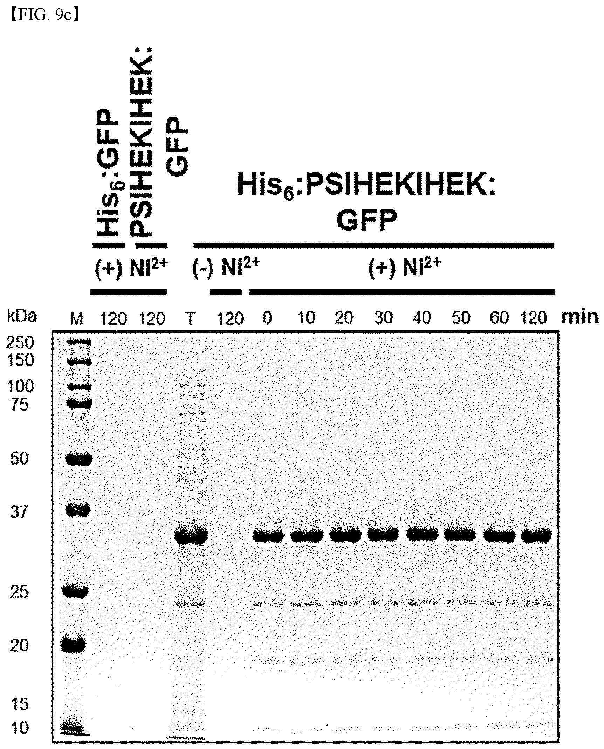

FIG. 9c is a diagram analyzing the degree of formation of a macromolecular self-assembly depending on the treatment time of cation Ni²⁺ selected as a specific inducer under the condition where ammonium sulfate is present as salt, by fusing a combination of a His tag and a PSIHEKIHEK peptide tag as purification tags to GFP.

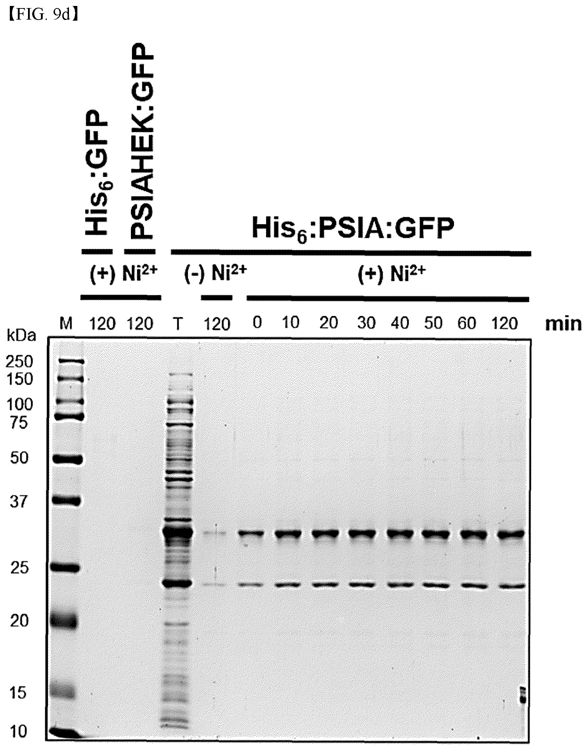

FIG. 9d is a diagram analyzing the degree of formation of a macromolecular self-assembly depending on the treatment time of cation Ni²⁺ selected as a specific inducer under the condition where ammonium sulfate is present as salt, by fusing a combination of a His tag and a PSIA peptide tag as purification tags to GFP.

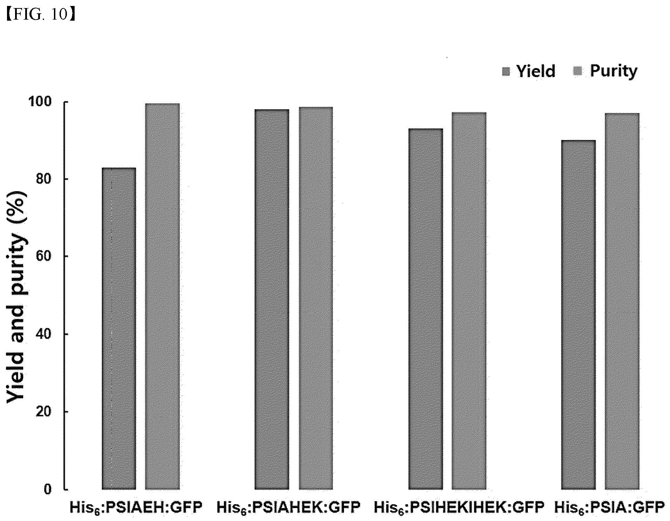

FIG. 10 is a diagram analyzing the yield (recovery rate) and purity by each unit through the degree of formation of a macromolecular self-assembly depending on the treatment time of cation Ni²⁺ selected as a specific inducer under the condition where ammonium sulfate is present as salt, by fusing a combination of a His tag and each of PSIAEH, PSIAHEK, PSIHEKIHEK and PSIA peptide tags alone as purification tags to GFP.

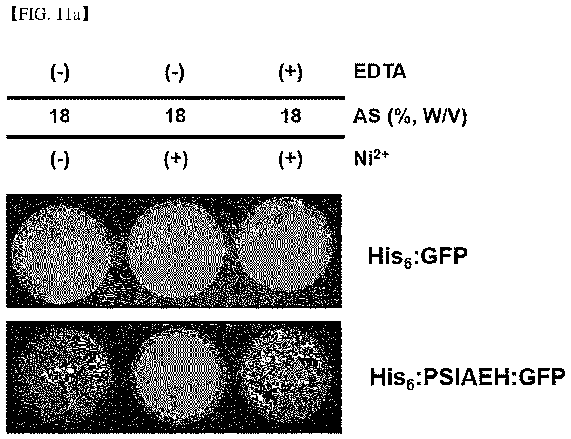

FIG. 11a is a diagram analyzing the possibility of filtration using a filter and recovery of a recombinant protein through irradiation of UV, when cation Ni²⁺ selected as a specific inducer is treated under the condition where ammonium sulfate is present as salt, by fusing a His tag alone, or a combination of a His tag and an PSIAEH peptide tag as purification tags to GFP.

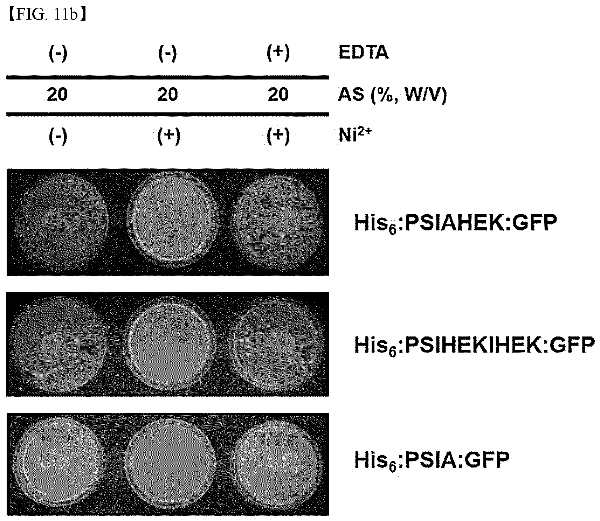

FIG. 11b is a diagram analyzing the possibility of filtration using a filter and recovery of a recombinant protein through irradiation of UV, when cation Ni²⁺ selected as a specific inducer is treated under the condition where ammonium sulfate is present as salt, by fusing a combination of a His tag and a PSIAHEK peptide tag, a combination of a His tag and a PSIHEKIHEK peptide tag or a combination of a His tag and an PSIA peptide tag as purification tags to GFP.

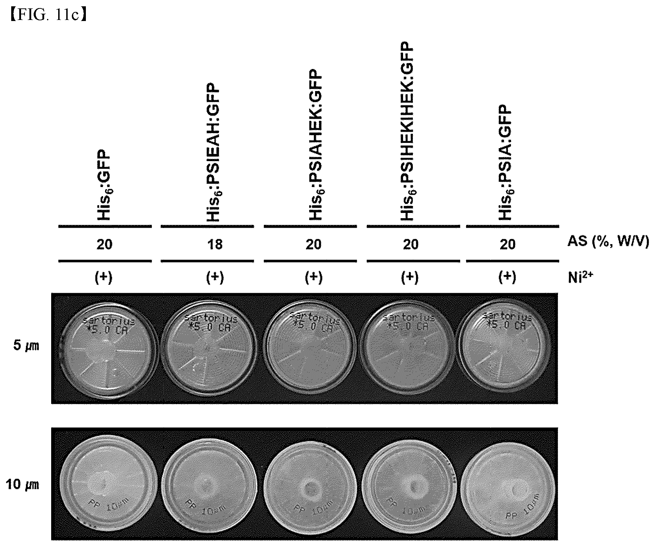

FIG. 11c is a diagram analyzing the possibility of filtration using a filter and recovery of a recombinant protein through irradiation of UV, when cation Ni²⁺ selected as a specific inducer is treated under the condition where ammonium sulfate is present as salt, by fusing a combination of a His tag and a PSIAHEK peptide tag, a combination of a His tag and a PSIHEKIHEK peptide tag or a combination of a His tag and an PSIA peptide tag as purification tags to GFP.

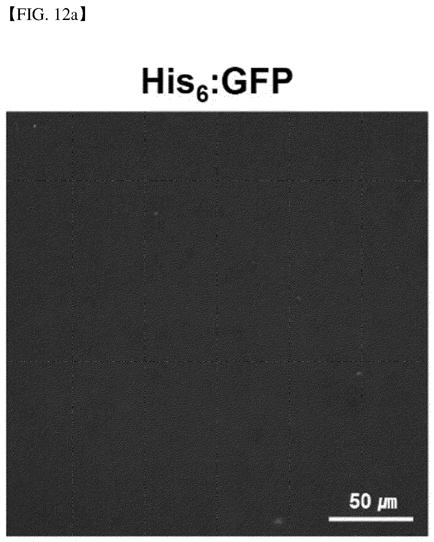

FIG. 12a is an image obtained under the condition where cation Ni²⁺ selected as a specific inducer is treated on condition that ammonium sulfate is present as salt to His₆:GFP, to analyze the formation of a macromolecular self-assembly and the size thereof through a fluorescence microscope.

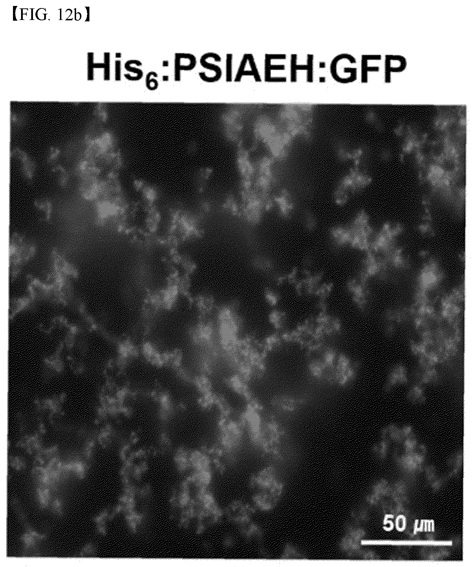

FIG. 12b is an image obtained under the condition where cation Ni²⁺ selected as a specific inducer is treated on condition that ammonium sulfate is present as salt to His₆:PSIAEH:GFP, to analyze the formation of a macromolecular self-assembly and the size thereof through a fluorescence microscope.

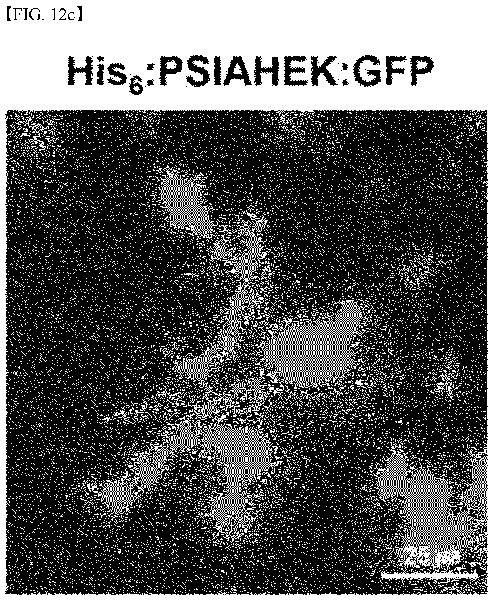

FIG. 12c is an image obtained under the condition where cation Ni²⁺ selected as a specific inducer is treated on condition that ammonium sulfate is present as salt to His₆:PSIAHEK:GFP, to analyze the formation of a macromolecular self-assembly and the size thereof through a fluorescence microscope.

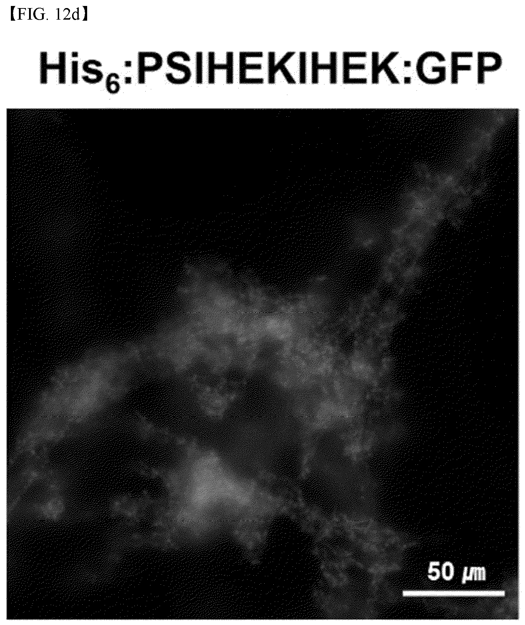

FIG. 12d is an image obtained under the condition where cation Ni²⁺ selected as a specific inducer is treated on condition that ammonium sulfate is present as salt to His₆:PSIHEKIHEK:GFP, to analyze the formation of a macromolecular self-assembly and the size thereof through a fluorescence microscope.

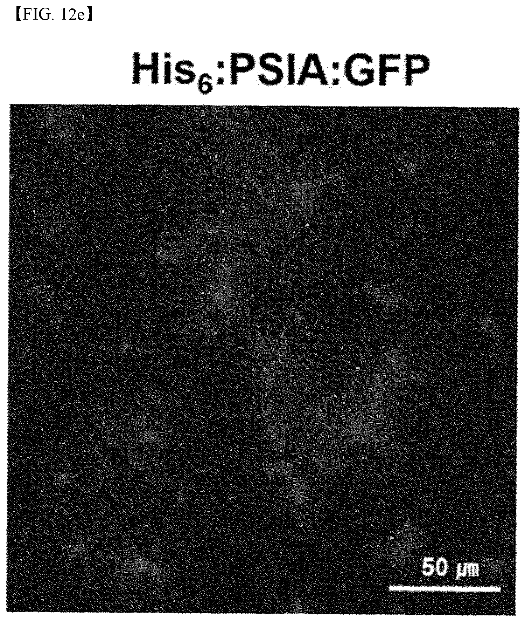

FIG. 12e is an image obtained under the condition where cation Ni²⁺ selected as a specific inducer is treated on condition that ammonium sulfate is present as salt to His₆:PSIA:GFP, to analyze the formation of a macromolecular self-assembly and the size thereof through a fluorescence microscope.

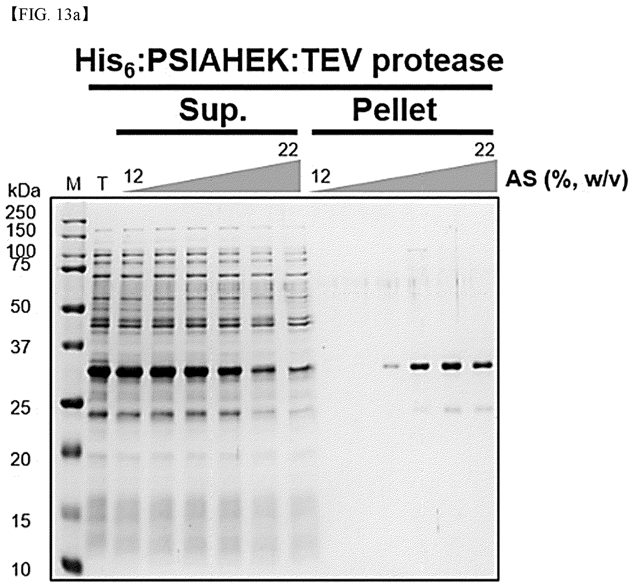

FIG. 13a is a diagram analyzing the degree of formation of a macromolecular self-assembly and the applicability to a recombinant protein other than GFP, when cation Ni²⁺ selected as a specific inducer is treated under the condition where various concentrations of ammonium sulfate are present as salt, by fusing a combination of a His tag and a PSIAHEK peptide tag as purification tags to TEV protease.

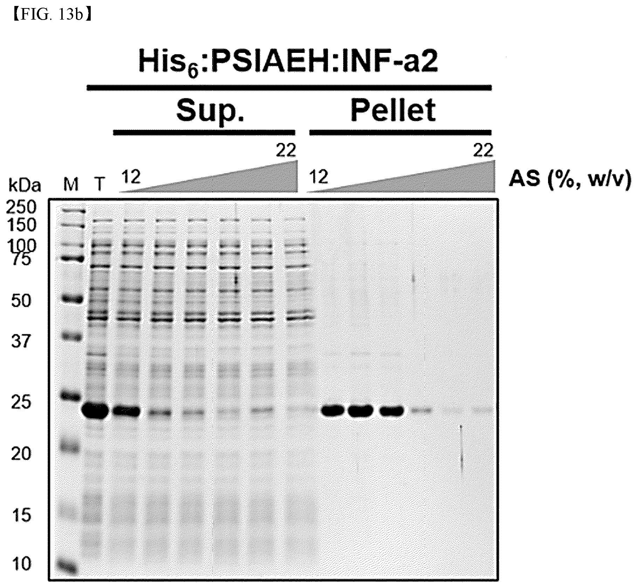

FIG. 13b is a diagram analyzing the degree of formation of a macromolecular self-assembly and the applicability to a recombinant protein other than GFP, when cation Ni²⁺ selected as a specific inducer is treated under the condition where various concentrations of ammonium sulfate are present as salt, by fusing a combination of a His tag and a PSIAEH peptide tag as purification tags to interferon alpha 2.

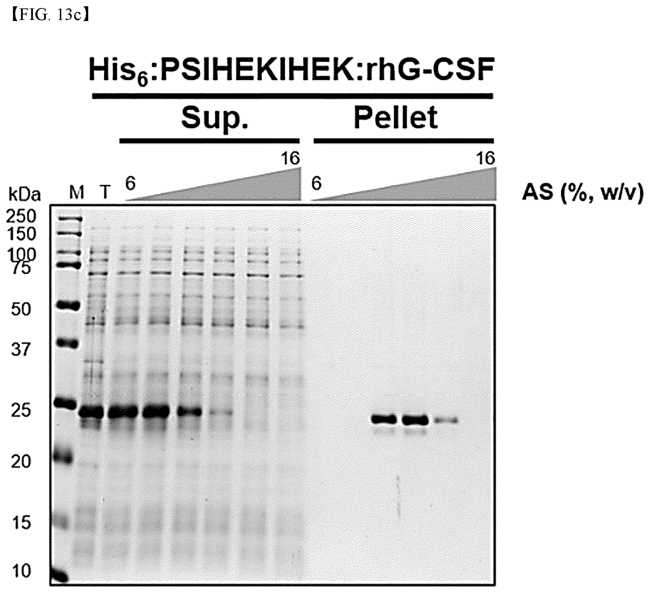

FIG. 13c is a diagram analyzing the degree of formation of a macromolecular self-assembly and the applicability to a recombinant protein other than GFP, when cation Ni²⁺ selected as a specific inducer is treated under the condition where various concentrations of ammonium sulfate are present as salt, by fusing a combination of a His tag and a PSIHEKIHEK peptide tag as purification tags to granulocyte macrophage colony stimulating factor.

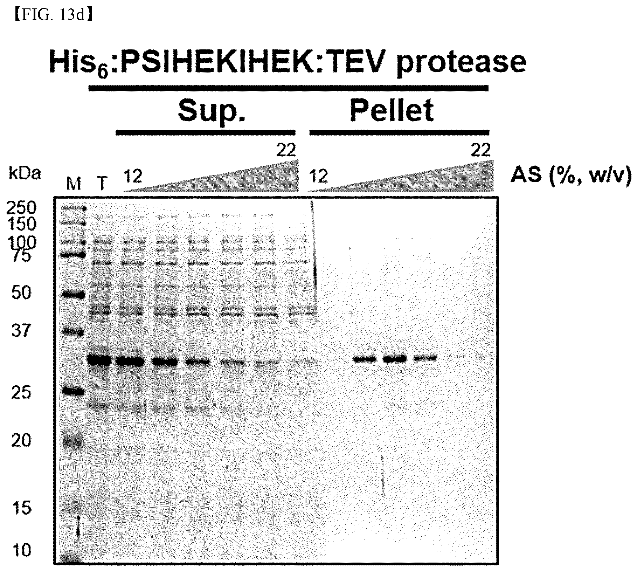

FIG. 13d is a diagram analyzing the degree of formation of a macromolecular self-assembly and the applicability to a recombinant protein other than GFP, when cation Ni²⁺ selected as a specific inducer is treated under the condition where various concentrations of ammonium sulfate are present as salt, by fusing a combination of a His tag and a PSIHEKIHEK peptide tag as purification tags to TEV protease.

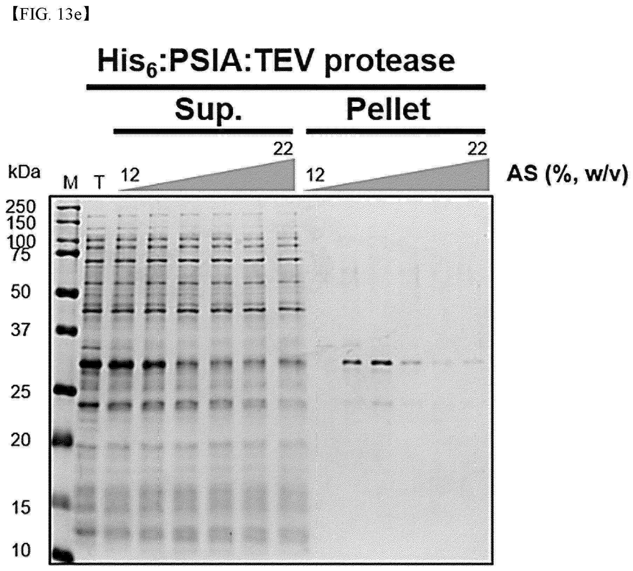

FIG. 13e is a diagram analyzing the degree of formation of a macromolecular self-assembly and the applicability to a recombinant protein other than GFP, when cation Ni²⁺ selected as a specific inducer is treated under the condition where various concentrations of ammonium sulfate are present as salt, by fusing a combination of a His tag and a PSIA peptide tag as purification tags to TEV protease.

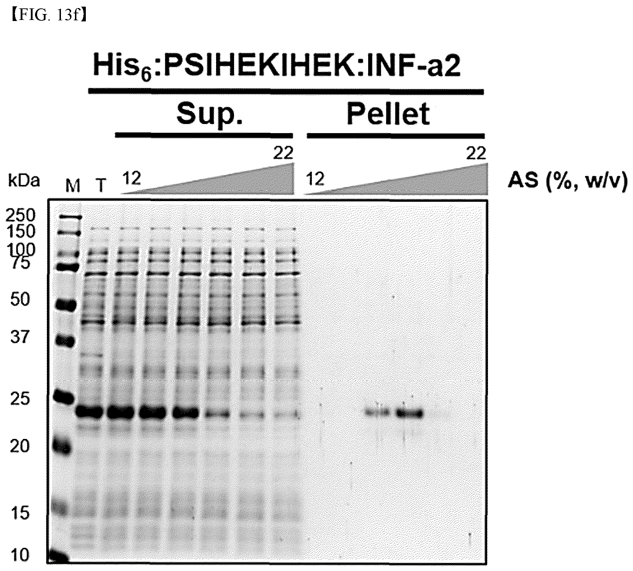

FIG. 13f is a diagram analyzing the degree of formation of a macromolecular self-assembly and the applicability to a recombinant protein other than GFP, when cation Ni²⁺ selected as a specific inducer is treated under the condition where various concentrations of ammonium sulfate are present as salt, by fusing a combination of a His tag and an PSIHEKIHEK peptide tag as purification tags to interferon alpha 2.

### [MODE FOR INVENTION]

Hereinafter, preferable embodiments of the present invention will be described with reference to attached drawings. However, the embodiments of the present invention may be modified into various other forms, and the scope of the present invention is not limited to the embodiments described below. In other words, it should be understood that all applications and alterations, equivalents and substitutes comprised in the concept and technology of the present invention are included.

### [Experimental materials and methods]

### <Experimental materials>

Ammonium sulfate ((NH₄)₂SO₄, CAT NO. A4915) and nickel chloride (NiCl₂, CAT NO. 339350) were purchased from Sigma Aldrich (St. Louis, MO, USA) as reagents of ACS reagent grade and 98% or more, respectively, and EDTA disodium salt dihydrate (C₁₀H₁₄N₂Na₂O₈·2H₂O, CAT NO. 0105) was purchased as biotechnology grade from VWR Life Science (Radnor, PA, USA) and used.

### Example 1. Construction of vectors for expressing protein

In order to establish a model system using GFP for constructing a recombinant protein purification system in a bead-free type self-assembly induction method, a GFP gene was amplified using F-Kpn I-Hinge-Bgl II-TEV cleavage site:GFP/R-Xho I, Hind III:GFP primers and cleaved with restriction enzyme, and then inserted into the Kpn I/Xho I site of the pET 28a vector to construct a pET 28a-GFP subvector. Additionally, by performing overlapping PCR using F-Nco I, BamH I-His₆/R-Kpn I-His₆ primers and then cleaving with restriction enzyme, and inserting into the Nco I/Kpn I site of the pET 28a-GFP subvector, a basic vector, pET 28a-His₆:GFP vector was completed. After that, based on the primer combinations of F-Nco I,BamH I-His₆:PSIAEH/R-Kpn I-PSIAEH; F-Nco I,BamH I-His₆:PSIAHEK/R-Kpn I-PSIAHEK; F-Nco I,BamH I-His₆:PSIHEKIHEK/R-Kpn I-PSIHEKIHEK; F-Nco I,BamH I-His₆:PSIA/R-Kpn I-PSIA, pET 28a-His₆:PSIAEH:GFP, pET 28a-His₆:PSIAHEK:GFP, pET 28a-His₆:PSIHEKIHEK:GFP, pET 28a-His₆:PSIA:GFP vectors were produced, respectively. In order to construct vectors except for the His₆ tag and use them as a control group, based on the primer combinations of -Nco I,BamH I-PSIAEH/R-Kpn I-PSIAEH; F-Nco I,BamH I-PSIAHEK/R-Kpn I-PSIAHEK; F-Nco I,BamH I-PSIHEKIHEK/R-Kpn I-PSIHEKIHEK, representative vectors of pET 28a-PSIAEH:GFP, pET 28a-PSIAHEK:GFP, pET 28a-PSIHEKIHEK:GFP were produced. In order to evaluate the purification expandability for various protein pharmaceuticals other than GFP, by performing PCR with the primer combinations of F-Bgl II-rhG-CSF/R-Hind III-rhG-CSF; F-Bgl II-INF-a2/R-Hind III-INF-a2; F-Bgl II-TEV protease/R-Hind III-TEV protease, and then cleaving them with Bgl II/Hind III restriction enzyme, and inserting them into the GFP site of the pET 28a-His₆:PSIAEH:GFP, pET 28a-His₆:PSIAHEK:GFP, pET 28a-His₆:PSIHEKIHEK:GFP, pET 28a-His₆:PSIA:GFP vectors, finally, representative vectors of pET 28a-His₆:PSIAHEK:TEV protease, pET 28a-His₆:PSIAEH:INF-a2, pET 28a-His₆:PSIHEKIHEK:rhG-CSF, pET 28a-His₆:PSIHEKIHEK:TEV protease, pET 28a-His₆:PSIA:TEV protease, pET 28a-His₆:PSIHEKIHEK:INF-a2 were completed. Specific sequence information of each of the tags and designed recombinant proteins was as Table 1 to Table 3 below.

**[Table 1]**

| Amino acid sequence and nucleic acid sequence information of the first polypeptide tag | | | | |
|---|---|---|---|---|
| Tag name | Amino acid sequence (N → C) | SEQ ID NO: | Nucleic acid sequence (5' → 3') | SEQ ID NO: |
| His₆ | HHHHHH | 1 | CATCACCATCACCACCAT | 21 |

**[Table 2]**

| Amino acid sequence and nucleic acid sequence information of the amorphous polypeptide tag (or the second polypeptide tag) | | | | |
|---|---|---|---|---|
| Tag name | Amino acid sequence (N → C) | SEQ ID NO: | Nucleic acid sequence (5' → 3') | SEQ ID NO: |
| PSIAEH | PSIAEHPSIAEHPSIAEH | 2 | CCAAGTATTGCTGAGCAT CCTAGCATAGCAGAACA CCCAAGTATAGCAGAGC AT | 22 |
| PSIAHEK | PSIAHEKPSIAHEKPSI AHEK | 3 | CCAAGTATTGCTCATGAA AAGCCTAGCATAGCACA CGAGAAGCCAAGTATAG CACATGAGAAA | 23 |
| PSIHEKI HEK | PSIHEKIHEKPSIHEKIHEKIH EKPSIHEKIHEK | 4 | CCAAGTATTCATGAAAA GATCCACGAGAAGCCTA GCATCCACGAGAAGATT CATGAGAAGCCGAGTAT ACACGAAAAGATTCACG AGAAG | 24 |
| PSIA | PSIAPSIAPSIA | 5 | CCAAGTATTGCTCCTAGC ATAGCACCAAGTATAGC A | 25 |

**[Table 3]**

| Amino acid sequence and nucleic acid sequence information of each fusion polypeptide, target protein and hinge region. | | | | |
|---|---|---|---|---|
| Clone name | Amino acid sequence (N → C) | SEQ ID NO: | Nucleic acid sequence (5' → 3') | SEQ ID NO: |
| His₆: PSIA EH | MDPMHHHHHHPSIAE HPSIAEHPSIAEH | 6 | ATGGATCCAATGCATCAC CATCACCACCATCCAAGT ATTGCTGAGCATCCTAGC ATAGCAGAACACCCAAG TATAGCAGAGCAT | 26 |
| His₆: PSIA HEK | MDPMHHHHHHPSIAH EKPSIAHEKPSIAHEK | 7 | ATGGATCCAATGCATCAC CATCACCACCATCCAAGT ATTGCTCATGAAAAGCCT AGCATAGCACACGAGAA GCCAAGTATAGCACATG AGAAA | 27 |
| His₆: PSIH EKIH EK | MDPMHHHHHHPSIHE KIHEKPSIHEKIHEKPSI HEKIHEK | 8 | ATGGATCCAATGCATCAC CATCACCACCATCCAAGT ATTCATGAAAAGATCCAC GAGAAGCCTAGCATCCA CGAGAAGATTCATGAGA AGCCGAGTATACACGAA AAGATTCACGAGAAG | 28 |
| His₆: PSIA | MDPMHHHHHHPSIAPS IAPSIA | 9 | ATGGATCCAATGCATCAC CATCACCACCATCCAAGT ATTGCTCCTAGCATAGCA CCAAGTATAGCA | 29 |
| PSIA EH | MDPMPSIAEHPSIAEHP SIAEH | 10 | ATGGATCCAATGCCAAGT ATTGCTGAGCATCCTAGC ATAGCAGAACACCCAAG TATAGCAGAGCAT | 30 |
| PSIA HEK | MDPMPSIAHEKPSIAH EKPSIAHEK | 11 | ATGGATCCAATGCCAAGT ATTGCTCATGAAAAGCCT AGCATAGCACACGAGAA GCCAAGTATAGCACATG AGAAA | 31 |
| PSIH EKIH EK | MDPMPSIHEKIHEKIHEKPSI HEKIHEKPSIHEKIHEK | 12 | ATGGATCCAATGCCAAGT ATTCATGAAAAGATCCAC GAGAAGCCTAGCATCCA CGAGAAGATTCATGAGA AGCCGAGTATACACGAA AAGATTCACGAGAAG | 32 |
| His₆:PSI AHEK: TEV protease | MDPMHHHHHHPSIAHE KPSIAHEKPSIAHEKGTG SPPVPSTPPTPSPSCRSGE SLFKGPRDYNPISSTICHL TNESDGHTTSLYGIGFGP FIITNKHLFRRNNGTLLV QSLHGVFKVKNTTTLQQ HLIDGRDMIIIRMPKDFP PFPQKLKFREPQREERIC LVTTNFQTKSMSSMVSD TSCTFPSSDGIFWKHWIQ TKDGQCGSPLVSTRDGFI VGIHSASNFTNTNNYFTS VPKNFMELLTNQEAQQ WVSGWRLNADSVLWG GHKVFMVKPEEPFQPVK EATQLMN | 13 | ATGGATCCAATGCATCAC CATCACCACCATCCAAGT ATTGCTCATGAAAAGCCT AGCATAGCACACGAGAA GCCAAGTATAGCACATGA GAAAGGTACCGGTTCTCC ACCAGTACCAAGCACACC TCCAACTCCGAGTCCGAG TTGTAGATCTGGAGAAAG CTTGTTTAAGGGGCCGCG TGATTACAACCCGATATC GAGCACCATTTGTCATTT GACGAATGAATCTGATGG GCACACAACATCGTTGTA TGGTATTGGATTTGGTCCC TTCATCATTACAAACAAG CACTTGTTTAGAAGAAAT AATGGAACACTGTTGGTC CAATCACTACATGGTGTAT TCAAGGTCAAGAACACC ACGACTTTGCAACAACAC CTCATTGATGGGAGGGAC ATGATAATTATTCGCATGC CTAAGGATTTCCCACCAT TTCCTCAAAAGCTGAAAT TTAGAGAGCCACAAAGG GAAGAGCGCATATGTCTT GTGACAACCAACTTCCAA ACTAAGAGCATGTCTAGC ATGGTGTCAGACACTAGT TGCACATTCCCTTCATCTG ATGGCATATTCTGGAAGC ATTGGATTCAAACCAAGG ATGGGCAGTGTGGCAGTC CATTAGTATCAACTAGAG ATGGGTTCATTGTTGGTAT ACACTCAGCATCGAATTT CACCAACACAAACAATTA TTTCACAAGCGTGCCGAA AAACTTCATGGAATTGTT GACAAATCAGGAGGCGC AGCAGTGGGTTAGTGGTT GGCGATTAAATGCTGACT CAGTATTGTGGGGGGGCC ATAAAGTTTTCATGGTGA AACCTGAAGAGCCTTTTC AGCCAGTTAAGGAAGCG ACTCAACTTATGAATTAA | 33 |
| His₆:PSI AEH:IN F-a2 | MDPMHHHHHHPSIAEHP SIAEHPSIAEHGTGSPPVP STPPTPSPSCRSENLYFQ GCDLPQTHSLGSRRTLM LLAQMRRISLFSCLKDR HDFGFPQEEFGNQFQKA ETIPVLHEMIQQIFNLFST KDSSAAWDETLLDKFYT ELYQQLNDLEACVIQGV GVTETPLMKEDSILAVR KYFQRITLYLKEKKYSPC AWEVVRAEIMRSFSLST NLQESLRSKE | 14 | ATGGATCCAATGCATCAC CATCACCACCATCCAAGT ATTGCTGAGCATCCTAGC ATAGCAGAACACCCAAG TATAGCAGAGCATGGTA CCGGTTCTCCACCAGTAC CAAGCACACCTCCAACTC CGAGTCCGAGTTGTAGAT CTGAAAACCTGTATTTTC AGGGCTGCGATCTCCCAC AAACCCACTCCTTAGGTT CTCGACGTACTCTAATGC TTCTCGCTCAGATGAGGA GGATTTCACTTTTCAGTT GTTTGAAGGATCGTCACG ACTTCGGATTTCCCCAAG AAGAGTTTGGAAACCAA TTCCAAAAGGCTGAGAC AATTCCAGTTTTGCACGA AATGATTCAACAAATCTT TAATCTGTTTAGCACTAA GGATAGCAGCGCAGCAT GGGATGAAACACTCCTTG ATAAATTTTATACAGAAT TATATCAGCAACTGAATG ACCTTGAGGCATGCGTCA TACAGGGTGTTGGGGTA ACAGAGACTCCTCTCATG AAAGAAGACAGTATTTT AGCCGTTCGTAAATATTT TCAGCGTATTACTCTTTA TCTTAAAGAAAAAAAAT ACTCCCCTTGCGCATGGG AGGTTGTCCGAGCAGAA ATTATGAGGAGCTTCTCC CTCTCTACAAATCTGCAA GAGTCTTTGAGAAGTAA AGAATGA | 34 |
| His₆:PSI HEKIH EK:rhG-CSF | MDPMHHHHHHPSIHEKI HEKPSIHEKIHEKPSIHEK IHEKGTGSPPVPSTPPTPS PSCRSAAAENLYFQGTP LGPASSLPQSFLLKCLEQ VRKIQGDGAALQEKLCA TYKLCHPEELVLLGHSL GIPWAPLSSCPSQALQLA GCLSQLHSGLFLYQGLL QALEGISPELGPTLDTLQ LDVADFATTIWQQMEEL GMAPALQPTQGAMPAF ASAFQRRAGGVLVASHL QSFLEVSYRVLRHLAQP | 15 | ATGGATCCAATGCATCAC CATCACCACCATCCAAGT ATTCATGAAAAGATCCAC GAGAAGCCTAGCATCCA CGAGAAGATTCATGAGA AGCCGAGTATACACGAA AAGATTCACGAGAAGGG TACCGGTTCTCCACCAGT ACCAAGCACACCTCCAA CTCCGAGTCCGAGTTGTA GATCTGCGGCCGCAGAA AACCTGTATTTTCAGGGC ACCCCCCTGGGCCCTGCC AGCTCCCTGCCCCAGAGC TTCCTGCTCAAGTGCTTA GAGCAAGTGAGGAAGAT CCAGGGCGATGGCGCAG CGCTCCAGGAGAAGCTG TGTGCCACCTACAAGCTG TGCCACCCCGAGGAGCT GGTGCTGCTCGGACACTC TCTGGGCATCCCCTGGGC TCCCCTGAGCAGCTGCCC CAGCCAGGCCCTACAGCT GGCAGGCTGCTTGAGCC AACTCCATAGCGGCCTTT TCCTCTACCAGGGGCTCC TACAGGCCCTGGAAGGG ATCTCCCCCGAGTTGGGT CCCACCTTGGACACACTA CAGCTGGACGTCGCCGA CTTTGCCACCACCATCTG GCAGCAGATGGAAGAAC TGGGAATGGCCCCTGCCC TACAGCCCACCCAGGGT GCCATGCCGGCCTTCGCC TCTGCTTTCCAGCGCCGG GCAGGAGGGGTCCTAGT TGCCTCCCATCTACAGAG CTTCCTGGAGGTGTCGTA CCGCGTTCTACGCCACCT TGCCCAGCCCTGA | 35 |
| His₆:PSI HEKIH EK:TE V protease | MDPMHHHHHHPSIHEKI HEKPSIHEKIHEKPSIHEK IHEKGTGSPPVPSTPPTPS PSCRSGESLFKGPRDYNP ISSTICHLTNESDGHTTSL YGIGFGPFIITNKHLFRRN NGTLLVQSLHGVFKVKN TTTLQQHLIDGRDMIIIR MPKDFPPFPQKLKFREPQ REERICLVTTNFQTKSMS SMVSDTSCTFPSSDGIFW KHWIQTKDGQCGSPLVS TRDGFIVGIHSASNFTNT NNYFTSVPKNFMELLTN QEAQQWVSGWRLNADS VLWGGHKVFMVKPEEP FQPVKEATQLMN | 16 | ATGGATCCAATGCATCAC CATCACCACCATCCAAGT ATTCATGAAAAGATCCAC GAGAAGCCTAGCATCCA CGAGAAGATTCATGAGA AGCCGAGTATACACGAA AAGATTCACGAGAAGGG TACCGGTTCTCCACCAGT ACCAAGCACACCTCCAA CTCCGAGTCCGAGTTGTA GATCTGGAGAAAGCTTGT TTAAGGGGCCGCGTGATT ACAACCCGATATCGAGC ACCATTTGTCATTTGACG AATGAATCTGATGGGCA CACAACATCGTTGTATGG TATTGGATTTGGTCCCTT CATCATTACAAACAAGC ACTTGTTTAGAAGAAATA ATGGAACACTGTTGGTCC AATCACTACATGGTGTAT TCAAGGTCAAGAACACC ACGACTTTGCAACAACAC CTCATTGATGGGAGGGA CATGATAATTATTCGCAT GCCTAAGGATTTCCCACC ATTTCCTCAAAAGCTGAA ATTTAGAGAGCCACAAA GGGAAGAGCGCATATGT CTTGTGACAACCAACTTC CAAACTAAGAGCATGTCT AGCATGGTGTCAGACACT AGTTGCACATTCCCTTCA TCTGATGGCATATTCTGG AAGCATTGGATTCAAACC AAGGATGGGCAGTGTGG CAGTCCATTAGTATCAAC TAGAGATGGGTTCATTGT TGGTATACACTCAGCATC GAATTTCACCAACACAA ACAATTATTTCACAAGCG TGCCGAAAAACTTCATGG AATTGTTGACAAATCAGG AGGCGCAGCAGTGGGTT AGTGGTTGGCGATTAAAT GCTGACTCAGTATTGTGG GGGGGCCATAAAGTTTTC ATGGTGAAACCTGAAGA GCCTTTTCAGCCAGTTAA GGAAGCGACTCAACTTAT GAATTGA | 36 |
| His₆:PSI A:TEV protease | MDPMHHHHHHPSIAPSI APSIAGTGSPPVPSTPPTP SPSCRSGESLFKGPRDYN PISSTICHLTNESDGHTTS LYGIGFGPFIITNKHLFRR NNGTLLVQSLHGVFKVK NTTTLQQHLIDGRDMIII RMPKDFPPFPQKLKFREP QREERICLVTTNFQTKS MSSMVSDTSCTFPSSDGI FWKHWIQTKDGQCGSPL VSTRDGFIVGIHSASNFT NTNNYFTSVPKNFMELL TNQEAQQWVSGWRLNA DSVLWGGHKVFMVKPE EPFQPVKEATQLMN | 17 | ATGGATCCAATGCATCAC CATCACCACCATCCAAGT ATTGCTCCTAGCATAGCA CCAAGTATAGCAGGTAC CGGTTCTCCACCAGTACC AAGCACACCTCCAACTCC GAGTCCGAGTTGTAGATC TGGAGAAAGCTTGTTTAA GGGGCCGCGTGATTACA ACCCGATATCGAGCACC ATTTGTCATTTGACGAAT GAATCTGATGGGCACAC AACATCGTTGTATGGTAT TGGATTTGGTCCCTTCAT CATTACAAACAAGCACTT GTTTAGAAGAAATAATG GAACACTGTTGGTCCAAT CACTACATGGTGTATTCA AGGTCAAGAACACCACG ACTTTGCAACAACACCTC ATTGATGGGAGGGACAT GATAATTATTCGCATGCC TAAGGATTTCCCACCATT TCCTCAAAAGCTGAAATT TAGAGAGCCACAAAGGG AAGAGCGCATATGTCTTG TGACAACCAACTTCCAAA CTAAGAGCATGTCTAGCA TGGTGTCAGACACTAGTT GCACATTCCCTTCATCTG ATGGCATATTCTGGAAGC ATTGGATTCAAACCAAG GATGGGCAGTGTGGCAG TCCATTAGTATCAACTAG AGATGGGTTCATTGTTGG TATACACTCAGCATCGAA TTTCACCAACACAAACAA TTATTTCACAAGCGTGCC GAAAAACTTCATGGAATT GTTGACAAATCAGGAGG CGCAGCAGTGGGTTAGT GGTTGGCGATTAAATGCT GACTCAGTATTGTGGGGG GGCCATAAAGTTTTCATG GTGAAACCTGAAGAGCC TTTTCAGCCAGTTAAGGA AGCGACTCAACTTATGAA TTGA | 37 |
| His₆:PSI HEKIH EK:INF -a2 | MDPMHHHHHHPSIHEKI HEKPSIHEKIHEKPSIHEK IHEKGTGSPPVPSTPPTPS PSCRSENLYFQGCDLPQT HSLGSRRTLMLLAQMRR ISLFSCLKDRHDFGFPQE EFGNQFQKAETIPVLHE MIQQIFNLFSTKDSSAAW DETLLDKFYTELYQQLN DLEACVIQGVGVTETPL MKEDSILAVRKYFQRITL YLKEKKYSPCAWEVVR AEIMRSFSLSTNLQESLR SKE | 18 | ATGGATCCAATGCATCAC CATCACCACCATCCAAGT ATTCATGAAAAGATCCAC GAGAAGCCTAGCATCCA CGAGAAGATTCATGAGA AGCCGAGTATACACGAA AAGATTCACGAGAAGGG TACCGGTTCTCCACCAGT ACCAAGCACACCTCCAA CTCCGAGTCCGAGTTGTA GATCTGAAAACCTGTATT TTCAGGGCTGCGATCTCC CACAAACCCACTCCTTAG GTTCTCGACGTACTCTAA TGCTTCTCGCTCAGATGA GGAGGATTTCACTTTTCA GTTGTTTGAAGGATCGTC ACGACTTCGGATTTCCCC AAGAAGAGTTTGGAAAC CAATTCCAAAAGGCTGA GACAATTCCAGTTTTGCA CGAAATGATTCAACAAA TCTTTAATCTGTTTAGCA CTAAGGATAGCAGCGCA GCATGGGATGAAACACT CCTTGATAAATTTTATAC AGAATTATATCAGCAACT GAATGACCTTGAGGCAT GCGTCATACAGGGTGTTG GGGTAACAGAGACTCCT CTCATGAAAGAAGACAG TATTTTAGCCGTTCGTAA ATATTTTCAGCGTATTAC TCTTTATCTTAAAGAAAA AAAATACTCCCCTTGCGC ATGGGAGGTTGTCCGAG CAGAAATTATGAGGAGC TTCTCCCTCTCTACAAAT CTGCAAGAGTCTTTGAGA AGTAAAGAATGA | 38 |
| GFP | SKGEELFTGVVPILVEL DGDVNGHKFSVSGEG EGDATYGKLTLKFICT TGKLPVPWPTLVTTFS YGVQCFSRYPDHMKR HDFFKSAMPEGYVQE RTIFFKDDGNYKTRAE VKFEGDTLVNRIELKGI DFKEDGNILGHKLEYN YNSHNVYIMADKQKN GIKANFKTRHNIEDGG VQLADHYQQNTPIGD GPVLLPDNHYLSTQSA LSKDPNEKRDHMVLL EFVTAAGITHGMDELY K | 19 | AGTAAAGGAGAGGAGTT GTTTACTGGTGTTGTCCC GATTTTAGTTGAACTTGA CGGTGATGTTAATGGGCA CAAGTTCTCTGTCAGTGG AGAAGGGGAAGGCGATG CAACATATGGTAAGCTCA CGTTGAAGTTTATTTGCA CTACTGGAAAACTCCCTG TTCCGTGGCCAACACTTG TGACTACGTTTTCTTACG GTGTTCAGTGTTTTTCAA GATACCCTGATCATATGA AGCGGCACGATTTCTTTA AGAGCGCGATGCCTGAG GGATACGTGCAGGAAAG AACCATCTTCTTCAAGGA CGACGGTAATTATAAGA CACGTGCTGAAGTTAAGT TCGAGGGAGACACCTTG GTGAATCGAATAGAACTT AAAGGAATCGATTTTAA GGAAGATGGAAACATTC TCGGCCACAAGTTGGAGT ACAACTACAACTCACATA ACGTATACATAATGGCA GATAAGCAAAAGAACGG CATCAAGGCAAACTTCA AGACCAGGCACAATATC GAGGATGGGGGTGTGCA ACTAGCTGATCATTATCA GCAAAATACTCCAATTGG TGATGGACCTGTCCTCTT ACCAGATAATCATTATCT GTCCACGCAATCTGCCCT GTCGAAGGACCCCAACG AAAAGAGAGACCATATG GTGCTTCTTGAGTTCGTA ACAGCTGCTGGGATTACA CATGGTATGGACGAGCT ATATAAGTGA | 39 |
| Hinge | GSPPVPSTPPTPSPSC | 20 | GGTTCTCCACCAGTACCA AGCACACCTCCAACTCCG AGTCCGAGTTGT | 40 |

### Example 2. Induction and extraction of expression of fusion proteins

The vectors for expressing fusion proteins produced in Example 1 were transformed into BL21 (DE3) *E. coli* and cultured in a 37°C LB medium (containing kanamycin 50 µg/mℓ). In order to induce overexpression of proteins, 1.0 mM IPTG (isopropyl-β) was added at O.D₆₀₀ value of 0.6 and it was additionally cultured at 18°C for 18 hours. The cultured *E. coli* was centrifuged at 4,000 rpm at 4°C for 15 minutes to obtain pellets. For extraction of the overexpressed fusion proteins, by sonication with HEPES buffer (HEPES; containing 50 mM HEPES buffer (pH 7.5), Triton X-100, protease inhibitor cocktail (Roche, Mannheim, GEU)) and then centrifugation at 13,000 rpm at 4°C for 15 minutes, the supernatant comprising the fusion proteins was obtained.

### Example 3. Induction and optimization of formation of macromolecular self-assemblies

### Example 3-1. Optimization of ammonium sulfate concentration as salt

For optimization of the ammonium sulfate concentration, Ni²⁺ at a concentration of 0.5 mM was treated into the supernatant obtained after centrifugation by treating ammonium sulfate in a range of 12~22% [w/v] at intervals of 2%.

### Example 3-2. Optimization of sodium chloride concentration as salt

In order to analyze the effect depending on the treatment concentration of sodium chloride as salt, Ni²⁺ at a concentration of 0.5 mM was treated under the condition of treatment of NaCl in a range of 0~3 M.

### Example 3-3. Optimization of buffer

For optimization of buffer, pH 7.4 HEPES buffer (HEPES; 50 mM HEPES buffer (pH 7.4)), pH 7.4 PBS buffer (PBS; 137 mM NaCl, 2.7 mM KCl, 10 mM Na₂HPO₄, 1.8 mM KH₂PO₄ (pH 7.4)), pH 7.4 PB buffer (PB; 10 mM Na₂HPO₄, 1.8 mM KH₂PO₄ pH 7.4)), pH 7.4 Tris buffer (Tris; 50 mM Tris-HCl buffer (pH 7.4)) were used, and 1% [v/v] TritonX-100 and 1X [v/v] Protease inhibitor cocktail tablets (Roche) were added to all and used. Ni²⁺ at a concentration of 0.5 mM was treated to the supernatant obtained by treating ammonium sulfate and then centrifuging under the buffer condition.

### Example 3-4. Optimization of Ni²⁺ concentration

For optimization of Ni²⁺ concentration, Ni²⁺ in a range of 0~2 mM was treated to the supernatant obtained by treating ammonium sulfate and then centrifuging.

### Example 3-5. Optimization of buffer pH

For optimization of buffer pH, the supernatant obtained by treating ammonium sulfate and centrifuging was used by adjusting the pH to a range of 5 to 10 using HCl and NaOH. Ni²⁺ at a concentration of 0.5 mM was treated into the supernatant obtained by treating ammonium sulfate and centrifuging under each pH condition.

### Example 3-6. Optimization of self-assembly formation reaction time

For optimization of the macromolecular self-assembly formation, it was measured immediately after treating Ni²⁺ at a concentration of 0.5 mM to the supernatant obtained by treating ammonium sulfate and then centrifuging, up to 60 minutes after the treatment at 10-minute intervals, and it was measured additionally up to 120 minutes.

### Example 4. Recovery of macromolecular self-assembly recombinant proteins and conversion into monomers

The recombinant proteins in which macromolecular self-assemblies were formed in the specific inducer or condition-dependent manner in Example 3 were recovered by a method such as centrifugation and filtration and the like.

Specifically, in the purification method by centrifugation, centrifugation was conducted at 3,000 rpm or more at 4°C for 10 minutes, and the macromolecular assembly recombinant proteins were obtained as pellets, and 5 mM EDTA was added to the same buffer used for the macromolecular assembly induction reaction to obtain recombinant proteins of monomers. In the filtration method, 0.2 and 5.0 µm syringe filters (Satorius, Goettingen, GEU), and a 10 µm syringe filter (Tisch, Ohio, USA) were used, and after filtering the recombinant proteins in which macromolecular self-assemblies were formed, a washing process was conducted using the same buffer used in the macromolecular assembly induction reaction. In addition, the macromolecular assemblies were converted into monomers or eluted using the same buffer in which a chelate such as EDTA was added. In order to visually confirm this, this was visually confirmed by irradiating the filters with a UV wavelength of 360 nm UV.

### Example 5. Confirmation of proteins purified by SDS-PAGE

The recombinant proteins of monomers recovered by centrifugation and filtration were taken in a certain amount, and SDS sample buffer (glycerol, 2M Tris-HCl(pH 6.8), 30% SDS, 2-mercaptoethanol) was added and they were heated to use for analysis, and the macromolecular self-assembly induction optimization condition search, purification yield and purity and the like of the purified proteins were evaluated by the general SDS-PAGE method.

### Example 6. Confirmation of formation of macromolecular self-assemblies through a fluorescence microscope

In order to analyze the formation of macromolecular self-assemblies and the approximate size and the like, a fluorescence microscope was used, and in order to detect a GFP fluorescence signal, a filter of excitation: 488 nm, emission: 520 nm was used.

### [Experimental results]

### Experimental example 1. Confirmation of inducers suitable for inducing formation of self-assemblies of fusion protein of a His tag and an amorphous peptide tag

In order to confirm the induction ability to form macromolecular self-assemblies in a specific inducer or condition-dependent manner, GFP was selected as a model protein, and by combining a His tag, and a PSIAEH peptide tag, a PSIAHEK peptide tag, a PSIHEKIHEK peptide tag and a PSIA peptide tag, which is an amorphous unit in which major amino acids are composed of charged and hydrophobic amino acids, as purification tags by the methods described in Example 1 and Example 2, His₆:PSIAEH:GFP, His₆:PSIAHEK:GFP, His₆:PSIHEKIHEK:GFP and His₆:PSIA:GFP fusion proteins were prepared. For a control group experiment, His₆:GFP fusion protein in which a His tag was fused alone, and PSIAEH:GFP, PSIAHEK:GFP, and PSIHEKIHEK:GFP fusion proteins that fused each of the amorphous or unstructured base units alone as a purification tag, were prepared (See FIG. 1b). Then, various cation inducers were treated to protein extracts comprising the fusion proteins to induce formation of macromolecular self-assemblies. The proteins in which formation of macromolecular self-assemblies was induced by the cation inducer were recovered by centrifugation and dissolved in the same buffer used during protein elution, and were confirmed by SDS-PAGE by adding SDS sample buffer and heating, and shown in FIG. 2a to 2h.

As a result, as shown in FIG. 2a to FIG. 2h, it was confirmed that the His₆:PSIAEH:GFP fusion protein induced formation of macromolecular self-assemblies by Ni²⁺, Co²⁺, Cu²⁺, Fe²⁺ (See FIG. 2c), and the His₆:PSIAHEK:GFP fusion protein induced formation of macromolecular self-assemblies excellently by Ni²⁺, Co²⁺, Cu²⁺, Zn²⁺, Fe²⁺ (See FIG. 2e). The His₆:PSIHEKIHEK:GFP fusion protein induced formation of macromolecular self-assemblies by Ni²⁺, Co²⁺, Cu²⁺, Ag²⁺, Zn²⁺, Fe²⁺ (See FIG. 2h), and the His₆:PSIA:GFP fusion protein induced formation of macromolecular self-assemblies by Cu²⁺, Zn²⁺, Fe²⁺ (See FIG. 2f).

On the other hand, in case of control groups using the His tag alone (His₆:GFP) or peptide tag alone (PSIAEH:GFP, PSIAHEK:GFP and PSIHEKIHEK:GFP), when the cation inducer such as Ni²⁺, Cu²⁺, Zn²⁺, Fe²⁺ and the like was treated, formation of macromolecular self-assemblies were not induced or were induced very weakly (See FIG. 2a, FIG. 2b, FIG. 2d and FIG. 2g).

To sum up the results, it was confirmed that the combination of the His tag and the peptide tag significantly increases the induction of formation of macromolecular self-assemblies by the various cation inducers. In the use of the combination of the His tag and all the peptide tags, the reactivity to the inducer Ni²⁺ was commonly very excellent, and in the subsequent experiment, Ni²⁺ was used as a specific inducer to progress the experiment.

### Experimental example 2. Confirmation of formation of self-assemblies when cation Ni²⁺ is treated as an inducer

Since it was confirmed that Ni²⁺ was suitable for forming self-assemblies as an inducer in Experimental example 1, using this, formation of self-assemblies of each His₆:PSIAEH:GFP, His₆:PSIAHEK:GFP, His₆:PSIHEKIHEK:GFP and His₆:PSIA:GFP fusion protein was confirmed when Ni²⁺ was treated.

Specifically, to each protein extract comprising the His₆:PSIAEH:GFP, His₆:PSIAHEK:GFP, His₆:PSIHEKIHEK:GFP and His₆:PSIA:GFP fusion proteins, His₆:GFP in which the His tag was fused alone, PSIAEH:GFP, PSIAHEK:GFP, and PSIHEKIHEK:GFP fusion proteins fused to each of the amorphous tags alone, obtained in Example 2, the cation inducer Ni²⁺ was treated at a concentration of 0 to 2 mM, respectively, to induce formation of macromolecular self-assemblies. The proteins in which formation of macromolecular self-assemblies was induced were recovered by centrifugation and dissolved in the same buffer used during protein elution, and were confirmed by SDS-PAGE by adding SDS sample buffer and heating, and the results were shown in FIG. 3a to 3d.

As a result, as shown in FIG. 3a to FIG. 3d, it was confirmed that the His₆:PSIAEH:GFP, His₆:PSIAHEK:GFP, His₆:PSIHEKIHEK:GFP and His₆:PSIA:GFP fusion proteins induced formation of macromolecular self-assemblies, whereas it was confirmed that the His₆: GFP in which His tag was fused alone, PSIAEH:GFP, PSIAHEK:GFP, and PSIHEKIHEK:GFP fusion proteins in which each amorphous tag was fused alone, did not induce formation of macromolecular self-assemblies in case of Ni²⁺ inducer treatment (See, FIG 3a to 3d).

### Experimental example 3. Establishment of optimal conditions for formation of macromolecular self-assemblies

For maximization of induction of macromolecular self-assemblies and optimization of high-purify purification conditions by removal of non-selective proteins other than the target protein and minimization of binding of the fusion proteins of the His tag and the amorphous peptide tag as purification tags, the effect of inducing formation of self-assemblies on various conditions such as buffer, salt treatment, concentration of Ni²⁺ treatment, pH change, treatment time, and the like was analyzed.

### Experimental example 3-1. Optimization of condition of ammonium sulfate concentration as salt

In order to confirm the condition of ammonium sulfate concentration suitable for formation of macromolecular self-assemblies of the fusion protein of the His tag and the amorphous peptide tag in which major amino acids were composed of charged and hydrophobic amino acids were fused as purification tags, the following experiment was performed.

Specifically, to each protein extract comprising the His₆:PSIAEH:GFP, His₆:PSIAHEK:GFP, His₆:PSIHEKIHEK:GFP and His₆:PSIA:GFP fusion proteins, the His₆:GFP in which the His tag was fused alone, and the PSIAEH:GFP, PSIAHEK:GFP and PSIHEKIHEK:GFP fusion proteins in which each peptide tag was fused alone, obtained in Example 2, formation of macromolecular self-assemblies was induced by the method described in Example 3-1. The proteins induced to form macromolecular self-assemblies were recovered by centrifugation, and dissolved in the same buffer used during protein elution, and were confirmed by SDS-PAGE by adding SDS sample buffer and heating, and the results were shown in FIG. 4a to FIG. 4h.

As a result, as shown in FIG. 4a to FIG. 4h, it was confirmed that formation of macromolecular self-assemblies was induced in a range of 14~22% (w/v) for the His₆:PSIAEH:GFP fusion protein, 16~22% (w/v) for the His₆:PSIAHEK:GFP fusion protein, 12~22% (w/v) for the His₆:PSIHEKIHEK:GFP fusion protein, 18~22% (w/v) for the His₆:PSIA fusion protein.

### Experimental example 3-2. Optimization of condition of sodium chloride concentration as salt

In order to confirm the condition of sodium chloride concentration suitable for formation of macromolecular self-assemblies of the fusion protein of the His tag and the amorphous peptide tag in which major amino acids were composed of charged and hydrophobic amino acids were fused as purification tags, the following experiment was performed.

Specifically, after inducing self-assemblies by the method described in Example 3-2 under the NaCl treatment condition in a 0-3 M concentration range as salt, they were confirmed by SDS-PAGE and the results were shown in FIG. 5a to FIG. 5d.

As a result, as shown in FIG. 5a to FIG. 5d, it was analyzed that the His₆:PSIAEH:GFP, and His₆:PSIA:GFP fusion proteins did not form macromolecular self-assemblies at all the NaCl concentrations, and formation of macromolecular self-assemblies was the most excellent when 100 mM NaCl was added for the His₆:PSIAHEK:GFP fusion protein and 150 mM NaCl was added for the His₆:PSIHEKIHEK:GFP fusion protein.

Through the results of Experimental examples 3-1 and 3-2, it was confirmed that induction of self-assemblies was relatively excellent when ammonium sulfate was treated compared to sodium chloride, so in subsequent experiments, the experiments were conducted using ammonium sulfate as salt.

### Experimental example 3-3. Optimization of buffer condition

In order to confirm the buffer suitable for formation of macromolecular self-assemblies of the fusion protein of the His tag and the amorphous peptide tag in which major amino acids were composed of charged and hydrophobic amino acids were fused as purification tags, the following experiment was performed.

Specifically, to each fusion protein extract, pH 7.4 HEPES buffer (HEPES; 50 mM HEPES buffer (pH 7.4)), pH 7.4 PBS buffer (PBS; 137 mM NaCl, 2.7 mM KCl, 10 mM Na₂HPO₄, 1.8 mM KH₂PO₄ (pH 7.4)), pH 7.4 PB buffer (PB; 10 mM Na₂HPO₄, 1.8 mM KH₂PO₄ pH 7.4)), pH 7.4 Tris buffer (Tris; 50 mM Tris-HCl buffer (pH 7.4)) were used as each buffer, and 1% [v/v] Triton X-100 and 1X [v/v] Protease inhibitor cocktail tablets (Roche) were added to all. Then, ammonium sulfate was treated as salt, and Ni²⁺ at a concentration of 0.5 mM was treated to induce self-assemblies. The results of analyzing the effect of inducing self-assemblies according to the type of buffer such as HEPES, Tris, phosphate and the like by SDS-PAGE were shown in FIG. 6a to FIG. 6d.

As a result, as shown in FIG. 6a to FIG. 6d, all fusion proteins showed the most excellent effect of inducing formation of macromolecular self-assemblies when using HEPES (pH 7.4) buffer solution.

### Experimental example 3-4. Optimization of Ni²⁺ concentration

In order to confirm the Ni²⁺ concentration condition suitable for formation of macromolecular self-assemblies of the fusion protein of the His tag and the amorphous peptide tag in which major amino acids were composed of charged and hydrophobic amino acids were fused as purification tags, the following experiment was performed.

Specifically, ammonium sulfate was treated as salt to the protein supernatant comprising each fusion protein, and the inducer, Ni²⁺ cation was treated in various ranges of 0 to 2 mM concentrations, respectively, to induce self-assemblies by the method described in Example 3-4, and then the effect of inducing self-assemblies was confirmed, and the results were shown in FIG. 7a to FIG. 7d.

As a result, as shown in FIG. 7a to FIG. 7d, it was confirmed that in case of the His₆:PSIAEH:GFP, His₆:PSIAHEK:GFP, His₆:PSIHEKIHEK:GFP and His₆:PSIA:GFP fusion proteins, when the cation inducer Ni²⁺ was treated at a concentration of 0.5 mM after ammonium sulfate was treated, almost all monomers present in the protein extract formed macromolecular self-assemblies within 20 minutes.

### Experimental example 3-5. Optimization of buffer pH

In order to confirm the pH condition of buffer suitable for formation of macromolecular self-assemblies of the fusion protein of the His tag and the amorphous peptide tag in which major amino acids were composed of charged and hydrophobic amino acids were fused as purification tags, the following experiment was performed.

Specifically, the supernatant obtained after treating ammonium sulfate and centrifuging was adjusted to a range of pH 5 to 10 using HCl and NaOH, and self-assemblies were induced by the method described in Example 3-5, and then confirmed by SDS-PAGE, and the results were shown in FIG. 8a to FIG. 8d.

As a result, as shown in FIG. 8a to FIG. 8d, in case of the His₆:PSIA:GFP fusion protein, induction of formation of macromolecular self-assemblies occurred normally at pH 7 to pH 8, but the induction of macromolecular self-assembly formation tended to decrease at more basic condition. It was confirmed that in other fusion proteins, induction of formation of macromolecular self-assemblies occurred normally under basic condition of pH 7 to pH 10, and precipitation of non-specific proteins occurred under acidic condition of pH 5 or less.

### Experimental example 3-6. Optimization of induction time of formation of self-assemblies of fusion protein of His tag and amorphous peptide tag

In order to confirm the induction time condition suitable for formation of macromolecular self-assemblies of the fusion protein of the His tag and the amorphous peptide tag in which major amino acids were composed of charged and hydrophobic amino acids were fused as purification tags, the following experiment was performed.

Specifically, to the protein supernatant comprising each fusion protein, ammonium sulfate was treated, and the inducer, Ni²⁺ cation was treated at a concentration of 0.5 mM. Up to 60 minutes after treating Ni²⁺ cation, at 10-minute intervals, self-assemblies were induced by the method described in Example 3-6, and additionally, they were induced up to 120 minutes, and then the effect of inducing self-assemblies was confirmed by SDS-PAGE, and the results were shown in FIG. 9a to FIG. 9d.

As a result, as shown in FIG. 9a to FIG. 9d, it was confirmed that each fusion protein, in which the His tag was combined with an amorphous base unit in which the major amino acids composition was composed of charged and hydrophobic amino acids, formed macromolecular self-assemblies within 20 minutes all.

### Experimental example 4. Confirmation of recovery of recombinant proteins induced to form macromolecular self-assemblies

### Experimental example 4-1. Confirmation of yield and purity of fusion protein of His tag and amorphous peptide tag

After inducing formation of self-assemblies of the fusion proteins of the His tag and the amorphous peptide tag under the condition optimized for formation of self-assemblies confirmed in Experimental example 3, they were recovered by a centrifugation method.

Specifically, the centrifugation method induced the formation of macromolecular self-assemblies with the optimal conditions and a cation inducer, followed by centrifugation at 3,000 rpm or more at 4°C for 10 minutes to recover the recombinant proteins in pellet form, and treating them with EDTA to convert multimers to monomers. The converted monomers were confirmed by SDS-PAGE, and the yield and purity were measured using Image Lab program of Bio-Rad company. The yield was calculated by the following formula (Equation 1) after inducing self-assemblies using ammonium sulfate and Ni²⁺. Yield = intensity of recovered target protein band/(intensity of unrecovered target protein band + intensity of recovered target protein band) × 100

The purity was expressed as a percentage by measuring the proportion that the purified protein bands among the total proteins accounted for.

As a result, as shown in FIG. 10, when 0.5 mM of the cation inducer Ni²⁺ was treated after treating ammonium sulfate, the maximum yield and maximum purity of each fusion protein of the His tag and the alpha-helix based peptide tag were analyzed to be 83%, 99% in the His₆:PSIAEH:GFP fusion protein, 98%, 98% in the His₆:PSIAHEK:GFP fusion protein, 90%, 97% in the His₆:PSIHEKIHEK:GFP fusion protein, 83%, 97% in the His₆:PSIA:GFP fusion protein.

### Experimental example 4-2. Confirmation of purification of fusion proteins of His tag and amorphous peptide tag using a filter

A method using a filter conducted a washing process using the same buffer used for the induction reaction of macromolecular assemblies after filtering the recombinant proteins in which the macromolecular self-assemblies were formed with the optimal condition and cation inducer to the protein extract using 0.2 and 5.0 µm syringe filters (Satorius, Goettingen, GEU), a 10 µm syringe filter (Tisch, Ohio, USA). In addition, the macromolecular assemblies were converted into monomers or eluted using the same buffer to which a chelate such as EDTA was added. In order to visually confirm this, the filter was irradiated with a 360 nm UV wavelength to visually confirm it.

As a result, as shown in FIG. 11a to FIG. 11c, in the filter of the protein solution to which ammonium sulfate and Ni²⁺ were treated, fluorescence could be observed by irradiating UV, as the macromolecular self-assemblies were filtered through the filter. After that, when the EDTA-added buffer was passed through the filter, the recombinant protein multimers were converted into monomers by EDTA and eluted, so the filter did not exhibit fluorescence even when UV was irradiated. Like this, the conversion from multimers into monomers occurring by EDTA is reversible and a very rapid reaction that occurs within seconds. In addition, the process of removing the tag after purification can be also easily removed under a specific inducer or condition. Therefore, by using the present purification method, the recombinant proteins could be recovered easily and conveniently and quickly with a device and a material commonly available to anyone, and it is expected that they can be applied to a process of producing recombinant proteins to carry out scale-up without difficulty later.

### Experimental example 4-3. Confirmation of formation of macromolecular self-assemblies through a fluorescence microscope

In order to recover macromolecular self-assemblies by sedimentation by low-speed centrifugation or filtration through a *µ*m scale filtration filter, the assemblies should be formed to a size larger than that of bacteria. In order to visually analyze the formation and size of these macromolecular self-assemblies, they were observed under a fluorescence microscope. In the analysis, His₆:PSIAEH:GFP, His₆:PSIAHEK:GFP, His₆:PSIHEKIHEK:GFP and His₆:PSIA:GFP were used, and as a control group, His₆:GFP was used. As specific experimental conditions, the optimized purification conditions of Experimental example 3 and Experimental example 4 were applied.

At first, as a result of observing formation of macromolecular assemblies, in case of His₆:GFP, assemblies with a very small size were partially observed, but in case of His₆:PSIAEH:GFP, His₆:PSIAHEK:GFP, His₆:PSIHEKIHEK:GFP and His₆:PSIA:GFP, it could be observed that macromolecular self-assemblies were formed overall. Formation of these macromolecular self-assemblies is three-dimensional, so it is very difficult to measure the accurate size, but as a result of analyzing the size of the macromolecular self-assemblies based on a planar image on the basis of one focal plane, it could be confirmed that they were formed in a size of several hundreds *µ*m ~ mm (FIG. 12a to FIG. 12e). Such a size is a huge size reaching a few times ~ tens of the general size of bacteria, and is evaluated to be equal to or larger than the average size of plant cells classified as large in size among eukaryotic cells.

### Experimental example 5. Confirmation of possibility of purification of various target proteins

The biopharmaceutical market size including antibody new drugs, vaccines, protein novel drugs, and the like is growing rapidly, and it will be possible to secure price competitiveness in the pharmaceutical market, if recombinant proteins including these biopharmaceuticals are easily and rapidly purified to high purity. In other words, it is significantly important to expand the versatility by applying the purification method to various target proteins. Accordingly, it was confirmed that the method of purifying recombinant proteins in the specific inducer or condition-dependent self-assembly inducing method of the present invention could be applied to various target proteins such as medical proteins, enzymes, and antibodies. As target proteins, granulocyte colony-stimulating factor, interferon alpha 2 and TEV protease, purification of various kinds of target proteins was conducted, and the optimized purification method in Experimental example 3 and Experimental example 4 was used.

As a result, as shown in FIG. 13a to FIG. 13f, the fusion protein in which macromolecular self-assemblies were formed best depending on the target protein, and the maximum yield and the maximum purity of the target protein searched in the present invention were analyzed as 86%, 93% for the His₆:PSIAHEK:TEV protease, 95%, 95% for the His₆:PSIAEH:INF-a2, 83%, 98% for the His₆:PSIHEKIHEK:rhG-CSF, 80%, 98% for the His₆:PSIHEKIHEK:TEV protease, 74%, 95% for the His₆:PSIA:TEV protease, and 83%, 97% for the His₆:PSIHEKIHEK:INF-a2. In other words, it was confirmed that they could be purified with the optimal yield and purity by selecting them among various units presented in the present invention, in purification of numerous target proteins other than the target proteins searched in the present invention.

## Claims

1. A fusion polypeptide comprising a first polypeptide represented by the amino acid sequence of SEQ ID NO: 1, and a second polypeptide which is a peptide tag in which the composition of major amino acids is composed of charged and hydrophobic amino acids.

2. The fusion polypeptide according to claim 1, wherein the second polypeptide is represented by one amino acid sequence selected from the group consisting of SEQ ID NO: 2 to SEQ ID NO: 5.

3. The fusion polypeptide according to claim 1, wherein the first polypeptide and the second polypeptide are sequentially linked.

4. A fusion protein, comprising the fusion polypeptide of claim 1 or the single polypeptide of claim 2, and a target protein.

5. The fusion protein according to claim 4, wherein the target protein is linked to the N-terminus or C-terminus of the fusion polypeptide.

6. The fusion protein according to claim 5, further comprising a cleavage site of protease, a hinge region of an immunoglobulin, or both of them between the fusion polypeptide and the target protein.

7. A polynucleotide encoding the fusion polypeptide of any one claim of claim 1 to claim 6.

8. An expression vector comprising the polynucleotide of claim 7.

9. A host cell transformed with the expression vector of claim 8.

10. A method for purifying a target protein, comprising culturing a cell comprising a polynucleotide encoding the fusion polypeptide of any one claim of claim 4 to claim 6.

11. The method for purifying a target protein according to claim 10, after the culturing, further comprising inducing formation of a self-assembly of a fusion protein produced in the cell.

12. The method for purifying a target protein according to claim 11, wherein the inducing formation of a self-assembly comprises adding an inducer.

13. The method for purifying a target protein according to claim 12, wherein the inducer is a cation.

14. The method for purifying a target protein according to claim 13, wherein the inducer has a concentration of 0.1 to 2.0 mM.

15. The method for purifying a target protein according to claim 11, wherein the inducing formation of a self-assembly comprises adding a salt comprising ammonium sulfate.

16. The method for purifying a target protein according to claim 15, wherein the ammonium sulfate has a concentration of 10 to 25% (w/v).

17. The method for purifying a target protein according to claim 11, wherein the inducing formation of a self-assembly is performed under the condition of pH 6 to pH 10.

18. The method for purifying a target protein according to claim 11, further comprising selectively separating the formed self-assembly.

19. The method for purifying a target protein according to claim 18, wherein the separating is performed by a centrifugation method or a filtration method.

20. A composition for purifying a protein comprising a first polypeptide represented by the amino acid sequence of SEQ ID NO: 1, or a second polypeptide represented by an amino acid sequence selected from the group consisting of SEQ ID NO: 2 to SEQ ID NO: 5.

21. An amorphous peptide having ability to form a self-assembly in which major amino acids are composed of charged and hydrophobic amino acids.

22. The peptide according to claim 21, wherein the peptide is represented by one amino acid sequence selected from the group consisting of SEQ ID NO: 2 to SEQ ID NO: 5.
